(19) Europäisches Patentamt / European Patent Office / Office européen des brevets

(11) **EP 4 262 942 B1**

(12) **EUROPEAN PATENT SPECIFICATION**

(45) Date of publication and mention
of the grant of the patent:
**05.02.2025 Bulletin 2025/06**

(21) Application number: **21836198.8**

(22) Date of filing: **17.12.2021**

(51) International Patent Classification (IPC):
*A61M 15/00* (2006.01)  *A24F 40/05* (2020.01)
*A24F 40/20* (2020.01)  *A24F 40/42* (2020.01)
*A24F 42/20* (2020.01)  *A61M 15/06* (2006.01)

(52) Cooperative Patent Classification (CPC):
**A61M 15/0005; A24F 42/20; A61M 15/003;
A61M 15/0035; A61M 15/0036;** A61M 15/0021;
A61M 15/06; A61M 2202/064

(86) International application number:
**PCT/EP2021/086635**

(87) International publication number:
**WO 2022/129600 (23.06.2022 Gazette 2022/25)**

(54) **INHALER ARTICLE HAVING HOLLOW TUBULAR ELEMENT**

INHALATORARTIKEL MIT EINEM HOHLEN ROHRFÖRMIGEN ELEMENT

ARTICLE D'INHALATEUR AYANT UN ÉLÉMENT TUBULAIRE CREUX

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.12.2020 EP 20215843**

(43) Date of publication of application:
**25.10.2023 Bulletin 2023/43**

(73) Proprietor: **Philip Morris Products S.A.
2000 Neuchâtel (CH)**

(72) Inventor: **CAMPITELLI, Gennaro
2000 Neuchâtel (CH)**

(74) Representative: **Reddie & Grose LLP
The White Chapel Building
10 Whitechapel High Street
London E1 8QS (GB)**

(56) References cited:
WO-A1-2017/109626    WO-A1-2020/178714
US-A1- 2010 300 439    US-A1- 2015 136 131

**Description**

The present disclosure relates to an inhaler article comprising a hollow tubular element.

Inhaler articles, such as dry powder inhalers, are not always fully suitable to provide dry powder particles to the lungs at inhalation or air flow rates that are within conventional smoking regime inhalation or air flow rates. Dry powder inhalers may be complex to operate or may involve moving parts. Dry powder inhalers often strive to provide a suitable dry powder dose or capsule load in a single draw.

Some dry powder inhalers have a component for storing the dry powder, such as a capsule. The capsule may be located within the inhaler and can be activated by being pierced by a separate piercing element. Once the capsule has been activated, a consumer may draw on the mouth end (downstream end or proximal end) of the inhaler to generate an air flow through the inhaler so that the capsule rotates about itself. The agitation of the capsule within the inhaler article and the air flow pressure causes the release of dry powder from the pierced capsule. The released dry powder is carried by the air flow to the mouth of a user.

Some inhaler articles comprise a retaining segment located downstream of the capsule. The retaining or support segment is provided to retain the capsule within the inhaler. The retaining or support segment is provided to provide strength or structural strength to the inhaler article. For example, the retaining or support segment may be strong enough in the direction of the longitudinal axis of the inhaler article to prevent the retaining or support segment from deforming when the capsule is pushed against the retaining or support segment when the capsule is pierced by a separate piercing element. Or, for example, the retaining or support segment may be strong enough in the direction perpendicular to the longitudinal axis of the inhaler article to prevent the inhaler article from being crushed during normal use. The retaining segment may be hollow or porous to allow the dry powder to pass through.

The upstream end (distal end) of the retaining segment of capsule-containing inhaler articles sustains considerable longitudinal force during the activation process of the capsule. During such activation process, a piercing element extends into the upstream end of the inhaler article in order to contact and pierce the capsule located within the article. Upon first contact, the piercing element pushes the capsule against the upstream end of the retaining (or support) segment in order to successfully pierce the capsule. Therefore, the downstream components of the inhaler article, especially the retainer segment, should be relatively resistant to deformation in the longitudinal direction, and particularly under compression.

In addition, a consumer may hold the inhaler article in the region of the retaining segment. To facilitate activation of the capsule a consumer may exert considerable transverse force on the inhaler article in the region of the retaining segment. Therefore, the retaining segment should also be relatively resistant to deformation in the transverse direction, and particularly under compression.

A retaining segment with a relatively low porosity may possess sufficient strength to facilitate activation of the capsule. However, when a consumer draws on an inhaler article comprising such a retaining segment the resistance to draw of the inhaler article is relatively high. As a result, the consumer may not be able to adequately deplete the capsule and the doses drawn by the consumer are relatively small.

WO 2020/178714 A1 describes an inhaler article including a body and a capsule cavity defined within the body, wherein a capsule is disposed within the capsule cavity, the capsule containing particles comprising nicotine. The inhaler article comprises an end cap and a boundary element that bound the capsule cavity and that cooperate to contain the capsule longitudinally within the capsule cavity.

It would be desirable to provide an inhaler article that is cost-effective and fast to manufacture and that performs effectively to provide a satisfactory experience for inhaler article consumers. In addition, it would be desirable to provide an inhaler article having sufficient rigidity to enable piercing of the capsule located inside the inhaler article and to prevent pushing of the capsule out of the inhaler article when the capsule is pierced. It would be desirable to provide an inhaler article having sufficient rigidity to resist deforming or crushing of the inhaler article when the capsule is pierced. It would be desirable to provide an inhaler article having sufficient rigidity to resist deforming or crushing during manipulation of the inhaler article.

The invention is defined in independent claim 1. Preferred embodiments are disclosed in the dependent claims. The inhaler article comprises a cavity. The inhaler article comprises a capsule. The capsule is located in the cavity. The capsule contains dry powder. The inhaler article also comprises a hollow tubular element. The hollow tubular element is disposed downstream of the capsule. The hollow tubular element comprises a peripheral portion. The peripheral portion defines a hollow inner region of the hollow tubular element. The hollow tubular element also comprises a support element. The support element is formed from a sheet. The support element extends from a first point at the peripheral portion. The support element extends across the hollow inner region. The support element extends to a second point at the peripheral portion.

In contrast to prior art inhaler articles, the inhaler article of the present disclosure comprises a hollow tubular element having a support element extending from a first point at its peripheral portion across its hollow inner region to a second point at its peripheral portion. The support element acts to provide a support barrier for one or more components disposed upstream of the hollow tubular element. For example, the support element can act to provide a support barrier for the capsule located in the cavity. This can help to prevent or restrict downstream movement of one or more components disposed upstream of the hollow tubular element, such as the capsule. This may be particularly beneficial when a piercing element is used to pierce the capsule from the upstream end of the inhaler article. The support barrier may prevent the capsule from being pushed out of the capsule cavity when the capsule is pierced from the upstream end of the inhaler article.

Furthermore, because the support element is formed from a sheet and extends from a first point at the peripheral portion across the hollow inner region to a second point at the hollow inner region, the hollow tubular element can still retain a suitably sized opening for one or both of air and dry powder to flow through the hollow tubular element, for example from the capsule. This means that the hollow tubular element can have a suitably low resistance to draw. This also means that the hollow tubular element can have a suitably low filtration effect.

In addition, forming the support element from a sheet can provide flexibility in design of the support element and in particular, of where the support element provides its support barrier. This is because the flexibility of the sheet can enable it to be easily formed into a shape that is most suitable for providing a support barrier for one or more components disposed upstream of the hollow tubular element, such as the capsule. This is particularly important for an inhaler article having a dry powder containing capsule, which may be provided in a range of shapes, sizes or both shapes and sizes. Thus, the flexibility in design of the support element and of where the support element provides its support barrier can mean that the support element can be designed to provide effective support for the inhaler article in which it is provided. In addition, the support element can be provided in a conformation that can be manufactured efficiently.

As used herein, the term "hollow tubular element" is used to denote a generally elongate element defining a lumen or airflow passage along a longitudinal axis thereof. In particular, the term "tubular" will be used in the following with reference to a tubular element having a tubular body with a substantially cylindrical cross-section and defining at least one airflow conduit establishing an uninterrupted fluid communication between an upstream end of the tubular body and a downstream end of the tubular body. However, it will be understood that alternative geometries (for example, alternative cross-sectional shapes) of the tubular body may be possible.

As used herein, the term "longitudinal" refers to the direction corresponding to the main longitudinal axis of the inhaler article, which extends between the upstream and downstream ends of the inhaler article.

The terms "upstream" and "downstream" refer to relative positions of elements of the holder, inhaler article and inhaler systems described in relation to the direction of inhalation air flow as it is drawn through the inhaler article, holder and inhaler systems. "Downstream" is the mouth end. "Upstream" is distal to the mouth end.

The term "longitudinal" refers to the direction corresponding to the main longitudinal axis of the inhaler article or inhaler system, extending between the upstream and downstream ends. During use, air is drawn through the inhaler article in the longitudinal direction from the upstream end to the downstream end. The term "transverse" refers to the direction that is perpendicular to the longitudinal axis. Any reference to the "cross-section" of the inhaler article or a component thereof refers to the transverse cross-section unless stated otherwise. The term "length" denotes the dimension of a component of the inhaler article in the longitudinal direction. For example, it may be used to denote the dimension of the capsule or hollow tubular element in the longitudinal direction. The term "tangential" refers to a direction that is at an angle from the referenced direction. For example, a tangential angle is not parallel with the referenced direction.

The terms "proximal" and "distal" are used to describe the relative positions of components, or portions of components, of the inhaler article, holder or inhaler system. Holders or elements (such as a sleeve) forming the holder, according to the disclosure have a proximal end which, in use, receives an inhaler article and an opposing distal end which may be a closed end, or have an end closer to the proximal end of the holder. Inhaler articles, according to the disclosure have a proximal end. In use, the powder particles exit the proximal end of the inhaler article for delivery to a user. The inhaler has a distal end opposing the proximal end. The proximal end of the inhaler article may also be referred to as the mouth end or downstream end. A distal end of a component may correspond to the upstream end of such a component. A proximal end of a component may correspond to the downstream end of such a component.

Unless otherwise specified, the resistance to draw (RTD) of a component or the inhaler article is measured in accordance with ISO 6565-2015. The RTD refers the pressure required to force air through the full length of a component. The terms "pressure drop" or "draw resistance" of a component or article may also refer to the "resistance to draw". Such terms generally refer to the measurements in accordance with ISO 6565-2015 are normally carried out at under test at a volumetric flow rate of about 17.5 millilitres per second at the output or downstream end of the measured component at a temperature of about 22 degrees Celsius, a pressure of about 101 kPa (about 760 Torr) and a relative humidity of about 60%.

As used herein, the term "sheet" denotes a laminar element having a width and length substantially greater than the thickness thereof.

The inhaler article may comprise an upstream section. The upstream section may comprise a folded end. The inhaler article may comprise a downstream section located downstream of the upstream section. The downstream section may be spaced apart from the upstream section. The downstream section may comprise the hollow tubular element.

The inhaler article may comprise a cavity defined between the upstream section and the downstream section. The cavity may be configured to contain the capsule containing an inhalable material. The inhalable material may be dry powder. The cavity may be configured to be in fluid communication with the exterior of the inhaler article.

The Young's modulus (or elastic modulus) of the material of the filter segment may be greater than about 10 MPa. Unless otherwise specified, the Young's modulus of the filter segment material is measured in accordance with ASTM E111-17. The Young's modulus (or elastic modulus) of the material of the filter segment may be greater than about 20 MPa. The Young's modulus (or elastic modulus) of the material of the filter segment may be greater than about 30 MPa. The Young's modulus (or elastic modulus) preferably refers to the Young's modulus of the material of a component along the longitudinal axis, or direction, of the component.

The capsule may be defined by having a particular puncture strength (in Newtons). The puncture strength of the capsule refers to the particular piercing or puncture force (in Newtons) a piercing element or needle is required to exert on the capsule in order to pierce or activate the capsule. Methods for measuring the puncture strength of the capsule are known to the skilled person. For example, the puncture strength of the capsule may be measured in accordance with ASTM F1306-16. For example, the puncture strength of a sample capsule may be measured with a 3.2 mm (8 gauge) diameter piercing element or hemispherical probe.

The capsule may be pierced by inserting a piercing element through the upstream end of the inhaler article and into the capsule. The piercing element may be solid. The piercing element may be hollow. The piercing element may be a needle. The piercing element may be between 27 gauge (outer diameter = 0.42 mm) to 4 gauge (outer diameter = 5 mm). The piercing element may have a diameter in the range of from about 0.42 mm to about 0.9 mm. The piercing element may have a diameter in the range of from about 0.6 mm to about 0.9 mm. The piercing element may have a diameter may be in a range from about 0.6 mm to about 0.9 mm. The piercing element may have a diameter in a range from about 0.7 mm to about 0.9 mm. The piercing element may have a diameter in a range from about 0.75 mm to about 0.85 mm. The piercing element may have a diameter about 0.8 mm. The piercing element may have a bevelled piercing end. For example the piercing element may have a single cutting plane or bevelled edge defining a cutting plane. The piercing element may have a cutting plane angle between the longitudinal axis of the piercing element and the single cutting plane. The cutting plane angle may be in a range from about 25 degrees and about 35 degrees. Preferably the cutting plane angle is in a range from about 28 degrees and about 32 degrees. Preferably the cutting plane angle is about 30 degrees. Piercing elements having these diameters and these cutting plane angles have been found to require a force of about 5 Newtons or less to activate or pierce the capsule contained in the inhaler article described herein.

The force applied on the upstream end of the capsule by the piercing element when piercing the capsule is transferred to the hollow tubular element. When the piercing element pushes against the capsule, this force is transferred to the hollow tubular element. Therefore, the hollow tubular element should be strong enough to remain intact when this force is applied. In addition, the hollow tubular element should enable particles released from the capsule after it is pierced, and entrained in the airflow through the inhaler article, to be delivered to the mouthpiece of the inhaler article so that they can be delivered to a user.

The hollow tubular element may be configured to sustain a force of at least about 50% of the puncture strength of the capsule being applied to the upstream end of the hollow tubular element without deforming substantially. The hollow tubular element may be configured to substantially retain its structure upon the application of a longitudinal force of at least about 200% of the puncture strength of the capsule applied to the upstream end of the hollow tubular element. The hollow tubular element may be configured to withstand a force of up to about 200% of the puncture strength of the capsule being applied to the upstream end of the hollow tubular element in a longitudinal direction without deforming substantially. The hollow tubular element may be configured to sustain a force of up to about 100% of the puncture strength of the capsule being applied to the upstream end of the hollow tubular element without deforming substantially. The hollow tubular element may be configured to sustain a force of up to about 200% of the puncture strength of the capsule being applied to the upstream end of the hollow tubular element without deforming substantially. The hollow tubular element may be configured to sustain a force of at least 50% of the puncture strength of the capsule to about 100% of the puncture strength of the capsule being applied to the upstream end of the hollow tubular element without deforming substantially. The hollow tubular element may be configured to sustain a force of at least 50% of the puncture strength of the capsule to about 200% of the puncture strength of the capsule being applied to the upstream end of the hollow tubular element without deforming substantially. The hollow tubular element may be configured to sustain a force of between 50% and 200% of the force required to puncture the capsule with the piercing element. The hollow tubular element may be configured to sustain a force of between about 3 Newtons and about 10 Newtons.

The hollow tubular element may extend from the cavity to the downstream end of the inhaler article. In other words, the length of the downstream section of the inhaler article may be the same as the length of the hollow tubular element.

The length of the hollow tubular element may be greater than about 10 mm. The length of the hollow tubular element may be greater than about 15 mm. The length of the hollow tubular element may be greater than about 20 mm. The length of the hollow tubular element may be less than about 30 mm. The length of the hollow tubular element may be between about 10 mm and 30 mm. The length of the hollow tubular element may be between about 10 millimetres and about 20 millimetres.

Preferably, the length of the hollow tubular element is between about 15 mm and 20 mm. The length of the hollow tubular element may be about 17 mm.

The inhaler article may have an outer diameter in a range from about 6 mm to about 10 mm, or from about 7 mm to about 10 mm, or from about 7 mm to about 9 mm, or from about 7 mm to about 8 mm, or about 7.2 mm. The inhaler article may have a length (along the longitudinal axis) in a range from about 40 mm to about 100 mm, or from about 40 mm to about 80 mm, or about 40 mm to about 60 mm. Preferably, the length of the inhaler article is about 45 mm. Preferably, the length of the inhaler article is selected such that the mouthpiece end of the inhaler article protrudes from a holder of the inhaler system, which is described in more detail below.

The (distal, front or upstream) end of the inhaler article may have a folded end. The folded end may fold back to expose the capsule in the cavity prior to piercing the capsule contained in the cavity. The folded end may fold back when the inhaler article is inserted into a holder having complimentary features to enable the folding of the distal end of the inhaler article.

The inhaler article may be fitted into a holder. The piercing element may be provided by the holder. The piercing element may be inserted into the capsule and removed from the capsule by the operation of a spring in the holder. The piercing element, provided by the holder, may then be inserted into the inhaler article, piercing the capsule. The piercing element may then be retracted from the inhaler article, leaving the pierced capsule in the inhaler article.

The inhaler article may be removed from the holder. The inhaler article may remain in the holder. Powder may then be removed from the capsule located in the inhaler article by drawing air through inhaler article from an air inlet located at the upstream end of the inhaler article to the mouthpiece or downstream end of the inhaler article. When air is drawn through the inhaler article, from the upstream end of the inhaler article to the downstream end of the inhaler article, past the pierced capsule, particles are released from the capsule and entrained into the airflow passing through the inhaler article, delivering particles to the mouthpiece or downstream end of the inhaler article and to the user. The holder may provide a swirled airflow to the downstream end of the inhaler article to induce a rotational airflow around the capsule, enabling the capsule to rotate, and improving the release of particles from the capsule in the capsule cavity. The holder may provide a swirled airflow to the downstream end of the inhaler article to induce a rotational airflow around the capsule, agitating the capsule, and improving the release of particles from the capsule in the capsule cavity. The inhaler article may have an end plug to induce a rotational airflow around the capsule, agitating the capsule, and improving the release of particles from the capsule in the capsule cavity. The inhaler article may have an end plug to induce a rotational airflow around the capsule, rotating the capsule and improving the release of particles from the capsule in the capsule cavity.

The body of the inhaler article, or the "inhaler article", may have any suitable shape. The body of the inhaler article, or "inhaler article" may resemble a smoking article or conventional cigarette in size and shape. The inhaler article may have a substantially uniform outer diameter along the length of the inhaler article. The inhaler article may have a substantially uniform inner diameter along the length of the inhaler article. The inhaler article may have any suitable transverse cross-sectional shape. For example, the transverse cross-section may be circular, elliptical, square or rectangular. The inhaler article preferably has a circular cross-section that may be uniform along the length of the inhaler article, forming an elongated cylindrical body.

The inhaler article may comprise a hollow tube extending from the upstream end of the inhaler article to the hollow tubular element so that the end plug and the capsule may be located within the hollow tube. The hollow tube and the upstream end of the hollow tubular element may define the cavity. The downstream end of the hollow tube may abut the upstream end of the hollow tubular element. The hollow tube may be formed of a polymeric or cellulosic material, or any other suitable material. The inhaler article may be formed of a biodegradable material. Preferably, the inhaler article is formed of paperboard or cardboard. The hollow tube may be formed of a biodegradable material. Preferably, the hollow tube is formed of paperboard or cardboard. The hollow tube may have a uniform thickness along its length. The hollow tube may have a thickness in a range from about 1 mm to about 2 mm.

The inhaler article may comprise a filter wrapper circumscribing the hollow tubular element of the downstream section. The inhaler article may comprise a wrapping material, or inhaler article wrapper, circumscribing the hollow tube and the downstream section. The wrapping material may be formed from a biodegradable material. The wrapping material may be formed from a paper wrapper.

The hollow tube may comprise an upstream section comprising a folded end. The folded end may define a central channel. The central channel may include a first end defining an upstream boundary of the capsule cavity and a second opposing end defining the distal end of the inhaler article body. The second opposing end may define an open distal end of the inhaler article body. When the folded end is opened, the second opposing end may define an open distal end of the inhaler article body. The central channel may extend along the longitudinal axis of the inhaler article and define an opening at the distal end of the inhaler article that is coaxial with the longitudinal axis of the inhaler article.

Advantageously, the hollow tube may comprise an open aperture along the longitudinal axis and may not have an element blocking or occluding the open distal end of the inhaler article, in order to reduce the complexity of the inhaler article. The consumer may simply occlude or block the open distal end with a holder or the consumer's finger to direct inhalation air flow substantially through the air inlets on the inhaler article, once the capsule has been pierced.

Air flow through the inhaler article preferably enters the inhaler article through the inhaler article upstream end via air flow inlet channels or through the open distal end, and then along the longitudinal axis of the inhaler article, via the capsule cavity and hollow tubular element, to exit at the mouthpiece or downstream end of the inhaler article.

The central channel may have a uniform inner or open diameter extending from the capsule cavity to the open distal end or upstream-most end of the inhaler article. The central channel may have a diameter that is at least about 50%, or at least about 70%, or at least about 75% of a diameter of the inhaler article. The central channel of the may have a diameter that is in a range from about 50% to about 90% of a diameter of the capsule retained within the capsule cavity. The central channel may have a diameter in a range from about 3 mm to about 6.5 mm, or from about 4 mm to about 6 mm, or from about 5 mm to about 6 mm or about 5.5 mm. Alternatively, the central channel may have a diameter in a range from about 0.5 mm to about 2 mm. Such sizing of the central channel ensures that the capsule may not fall out of the inhaler article via the central channel.

Preferably, a capsule is retained within the capsule cavity.

As discussed above, an end plug or a holder may induce rotational air flow or swirling air flow as air is drawn through the air flow inlet channels of the end plug or the holder, and through the capsule cavity. Advantageously, this swirling air flow produced by the air flow inlet channels of the end plug or the holder is useful for effective depletion of the capsule during consumption, after the capsule has been pierced. Advantageously, the "swirling" effect may cause agitation or rotation of the capsule to provide a uniform entrainment of a portion or a fraction of nicotine particles from the capsule over two or more, or five or more, or ten or more inhalations or "puffs" by a user.

The inhalable material may comprise nicotine. Preferably, the capsule contains pharmaceutically active particles. The pharmaceutically active particles may comprise nicotine. The pharmaceutically active particles may have a mass median aerodynamic diameter of about 5 micrometres or less, or in a range from about 0.5 micrometres to about 4 micrometres, or in a range from about 1 micrometres to about 3 micrometres.

Advantageously, the inhaler article efficiently provides nicotine particles to the lungs at inhalation or air flow rates that are within conventional smoking regime inhalation or air flow rates. The inhaler article or system described herein may provide a dry powder to the lungs at inhalation or air flow rates that are within conventional smoking regime inhalation or air flow rates. A consumer may take a plurality of inhalations or "puffs" where each "puff" delivers a fractional amount of dry powder contained within a capsule contained within the capsule cavity. The inhaler article may have a form similar to a conventional cigarette and may mimic the ritual of conventional smoking. The inhaler article may be simple to manufacture and convenient to use by a consumer.

Air flow management through a capsule cavity of the inhaler article may cause a capsule contained therein to rotate during inhalation and consumption. The capsule may contain particles containing nicotine (also referred to as "nicotine powder" or "nicotine particles") and optionally particles comprising flavour (also referred to as "flavour particles"). Rotation of the pierced capsule may suspend and aerosolize the nicotine particles released from the pierced capsule into the inhalation air moving through the inhaler article. The flavour particles may be larger than the nicotine particles and may assist in transporting the nicotine particles into the lungs of the user while the flavour particles preferentially remain in the mouth or buccal cavity of the user. The nicotine particles and optional flavour particles may be delivered with the inhaler article at inhalation or air flow rates that are within conventional smoking regime inhalation or air flow rates.

The term "nicotine" refers to nicotine and nicotine derivatives such as free-base nicotine, nicotine salts and the like.

The term "flavourant" or "flavour" refers to organoleptic compounds, compositions, or materials that alter and are intended to alter the taste or aroma characteristics of nicotine during consumption or inhalation thereof.

According to an aspect of the present disclosure, there is provided an inhaler system comprising an inhaler article as described herein and a holder for receiving the inhaler article. The holder comprises a housing defining a housing cavity configured to receive the inhaler article. The holder comprises a piercing element configured to extend into the housing cavity and to pierce the capsule of the inhaler article.

The holder may comprise a piercing element extending into the housing cavity configured to pierce the capsule of the inhaler article.

The holder for an inhaler article may be combined with an inhaler article (described herein) containing a capsule for activating the inhaler article by piercing the capsule, providing reliable activation of the capsule (by puncturing the capsule with the piercing element of the holder) within the inhaler article, and releasing the particles contained inside the capsule and enabling the inhaler article to deliver the particles to a consumer. The holder is separate from the inhaler article, but the consumer may utilize both the inhaler article and the holder while consuming the particles released within the inhaler article. A plurality of these inhaler articles may be combined with a holder to form a system or kit. A single holder may be utilized on 10 or more, or 25 or more, or 50 or more, or 100 or more, inhaler articles to activate (puncture or pierce) a capsule contained within each inhaler article and provide reliable activation and optionally, a visual indication (marking), for each inhaler article of the activation of the inhaler article.

A holder for an inhaler article includes a housing comprising a housing cavity for receiving an inhaler article and a sleeve configured to retain an inhaler article within the housing cavity. The sleeve comprising a sleeve cavity and being movable within the housing cavity along the longitudinal axis of the housing. The sleeve comprises a first open end and a second opposing end. The first open end is configured to receive the distal end of the inhaler article. The second opposing end of the sleeve is configured to contact the distal end of the inhaler article. The sleeve second opposing end is configured to direct substantially all inhalation air to flow through the inhaler article via the at least one air inlet extending in a direction that is non-parallel to the central channel.

The holder may include an opening structure to receive a folded distal end of the inhaler article and fold the folded end back so that the folded flaps of the distal end of the inhaler article fold back into the inhaler article, exposing the capsule in the capsule cavity to the interior of the holder.

Preferably the piercing element is fixed to and extends from a housing inner surface. The piercing element may be configured to extend through the second opposing end of the sleeve and into the capsule cavity to pierce the capsule along a longitudinal axis of the housing. The piercing element may be a metal or rigid needle. The piercing element may form a single aperture through the capsule received in the capsule cavity. The piercing element may be configured to pass through an end plug or a hollow tube of the inhaler article, precisely the central channel thereof, and into the capsule cavity.

The holder may further include a spring element configured to bias the sleeve toward the open proximal end of the housing, and between relaxed and compressed positions. The spring element may be contained within the housing cavity (also referred to as inhaler article cavity) of the holder and be compressed as the movable sleeve and inhaler article move toward the piercing element. The spring element may be located between the sleeve and distal end of the housing and contact the sleeve and distal end of the housing. The spring element may be between the distal end of the sleeve and the distal end of the housing. The spring element may contact the distal end of the sleeve and the distal end of the housing. The spring element may be disposed about the piercing element. The spring element may be co-axial with the piercing element. The spring element may be a conical spring.

The spring element biases the inhaler article away from the piercing element. In use, a user may insert an inhaler article into the inhaler article cavity of the holder. By doing this, the spring may be compressed allowing the inhaler article to move towards the distal end of the inhaler article cavity. Eventually, the piercing element may penetrate a capsule disposed within the inhaler article. Once this happens, the user may release the inhaler article, allowing the spring to bias the inhaler article towards the proximal end of the inhaler article cavity and away from the piercing element. The user may then inhale on the proximal end of the inhaler article.

The sleeve may define a first air inlet zone comprising at least one air aperture through the sleeve. The first air inlet zone is proximate to a proximal end of the sleeve. The first air inlet zone is configured to allow air to flow from an inside of the sleeve to an air flow channel formed between the sleeve and the housing inner surface. The sleeve may comprise a second air inlet zone comprising at least one air aperture through the sleeve. The second air inlet zone is proximate to a distal end of the sleeve. The second air inlet zone is configured to allow air to flow from the air flow channel to an inside of the sleeve.

The holder may include a marking element that extends into the housing (or inhaler article) cavity. The marking element may be configured to mark the surface of an inhaler article. The marking element may extend orthogonally to the holder or inhaler article longitudinal axis. The marking element may be configured to mark the outer surface of an inhaler article in a mechanical manner. For example, the marking element may be configured to scratch, cut, abrade, score, fold, or bend the outer surface of the inhaler article. The marking element may have a sharp end configured to scratch the inhaler outer surface when received within the housing cavity. The marking element may apply a colour to the inhaler article outer surface when received within the housing cavity. The marking element may mark the inhaler article outer surface when the piercing element penetrates a capsule disposed within the inhaler article. Thus, indicating that the inhaler article has been activated and may be consumed by a user. This may also advantageously prevent a user trying to reuse an inhaler article which has already been previously activated.

The marking element may extend orthogonally to the holder or inhaler article longitudinal axis. The marking element may be formed of a rigid material configured to provide a visual indication that the marking element has contacted the inhaler outer surface. The marking element may be fixed to the holder housing. The marking element may form the alignment pin, as described above.

The marking element may extend though at least a portion of a thickness of the holder. The marking element may extend through the sleeve. The marking element may extend into the housing cavity and into the sleeve. The marking element may extend beyond the at least the sleeve a marking distance so that the marking element contacts the inhaler outer surface when the inhaler article is received within the housing cavity. The marking element may be aligned with and mate with an elongated slot of the sleeve.

The inhaler article described herein may be combined with the piercing element or the holder including a piercing element to deliver the nicotine particles from the capsule to a user. The piercing element or piercing device (or holder) may be separate from or not form a portion of the inhaler article. A plurality of the inhaler articles may be combined with the piercing element or the piercing device (or holder) to form a kit.

A method includes, inserting an inhaler article into the sleeve of the holder for an inhaler article, as described herein, until the distal end of the inhaler article contacts the second opposing end of the sleeve. A method includes, inserting an inhaler article into the sleeve of the holder for an inhaler article, as described herein, until a distal folded end of the inhaler article contacts complimentary features of the holder to fold the flaps of the folded end into the distal end of the inhaler article and into the interior of the distal end of the inhaler article. The inhaler article includes a body, the body extending along an inhaler longitudinal axis from a mouthpiece end to a distal end, a body length, and a capsule disposed within the inhaler article body. Then, moving the inhaler article and sleeve toward the piercing element until the piercing element pierces the capsule. Then, moving the sleeve away from the piercing element until the piercing element is removed from the pierced capsule. Then drawing air into the second opposing end of the sleeve of the holder to direct inhalation air flow into the air inlets on the inhaler article to form rotational or swirling air flow through the cavity of the inhaler article. This swirling inhalation air flow is transmitted into the capsule cavity while the inhaler article is disposed within the holder for an inhaler article. The swirling inhalation air flow rotates or agitates the capsule to release the particles contained therein. The particles become entrained into the airflow. The consumer inhales the particles. This may be repeated several times until the particles contained in the capsule are depleted. For example, the user may take several "puffs" to inhale the particles contained in the capsule. The consumed inhaler article may then be removed from the holder and discarded. Then a fresh inhaler article may be inserted into the holder and the method repeated.

A capsule may be sealed within the inhaler article prior to consumption. For transport and storage, the inhaler article may be contained within a sealed or airtight container or bag. The inhaler article may include one or more peelable seal layers to cover the one or more air inlet channels at the distal end or the air outlet at the mouthpiece end of the inhaler article. This may ensure the inhaler articles maintain appropriate hygiene and freshness or may prevent the capsule from drying out and becoming hard or friable.

The capsule may rotate about its longitudinal or central axis when air is drawn through the inhaler article. The capsule may be formed of an airtight material that substantially contains the particles inside the capsule. The capsule may be configured to be pierced or punctured by a piercing element when the capsule is within the capsule cavity. The piercing element may be separate from or combined with the inhaler article. The capsule may be formed of any suitable material. The capsule may be formed of a metallic or polymeric material that serves to keep contaminants out of the capsule but may be pierced or punctured by a piercing element prior to consumption to enable the release of the nicotine particles from within the capsule. The capsule may be formed of a polymer material. The polymer material may be hydroxypropylmethylcellulose (HPMC). The capsule may be any suitable size. The capsule may be a size 1 to size 4 capsule, or a size 3 capsule, or a size 3 capsule.

The capsule may contain pharmaceutically active particles comprising nicotine (also referred to as "nicotine powder" or "nicotine particles") and optionally particles comprising flavour (also referred to as "flavour particles). The capsule may contain a predetermined amount of nicotine particles and optional flavour particles. The capsule may contain enough nicotine particles to provide at least 2 inhalations or "puffs", or at least about 5 inhalations or "puffs", or at least about 10 inhalations or "puffs". The capsule may contain enough nicotine particles to provide from about 5 to about 50 inhalations or "puffs", or from about 10 to about 30 inhalations or "puffs". Each inhalation or "puff" may deliver from about 0.1 mg to about 3 mg of nicotine particles to the lungs of the user or from about 0.2 mg to about 2 mg of nicotine particles to the lungs of the user or about 1 mg of nicotine particles to the lungs of the user.

The nicotine particles may have any useful concentration of nicotine based on the particular formulation employed. The nicotine particles may have at least about 1%wt nicotine up to about 30%wt nicotine, or from about 2%wt to about 25%wt nicotine, or from about 3%wt to about 20%wt nicotine, or from about 4%wt to about 15%wt nicotine, or from about 5%wt to about 13%wt nicotine. Preferably, about 50 to about 150 micrograms of nicotine may be delivered to the lungs of the user with each inhalation or "puff".

The capsule may hold or contain at least about 5 mg of nicotine particles or at least about 10 mg of nicotine particles. The capsule may hold or contain less than about 900 mg of nicotine particles, or less than about 300 mg of nicotine particles, or less than 150 mg of nicotine particles. The capsule may hold or contain from about 5 mg to about 300 mg of nicotine particles or from about 10 mg to about 200 mg of nicotine particles.

When flavour particles are blended or combined with the nicotine particles within the capsule, the flavour particles may be present in an amount that provides the desired flavour to each inhalation or "puff" delivered to the user.

The nicotine particles may have any useful size distribution for inhalation delivery preferentially into the lungs of a user. The capsule may include particles other than the nicotine particles. The nicotine particles and the other particles may form a powder system.

The capsule may hold or contain at least about 5 mg of a dry powder (also referred to as a powder system) or at least about 10 mg of a dry powder. The capsule may hold or contain less than about 900 mg of a dry powder, or less than about 300 mg of a dry powder, or less than about 150 mg of a dry powder. The capsule may hold or contain from about 5 mg to about 300 mg of a dry powder, or from about 10 mg to about 200 mg of a dry powder, or from about 25 mg to about 100 mg of a dry powder.

The dry powder or powder system may have at least about 40%, or at least about 60%, or at least about 80%, by weight of the powder system comprised in nicotine particles having a particle size of about 5 micrometres or less, or in a range from about 1 micrometre to about 5 micrometres.

The particles comprising nicotine may have a mass median aerodynamic diameter of about 5 micrometres or less, or in a range from about 0.5 micrometres to about 4 micrometres, or in a range from about 1 micrometres to about 3 micrometres or in a range from about 1.5 micrometres to about 2.5 micrometres. The mass median aerodynamic diameter is preferably measured with a cascade impactor.

The particles comprising flavour may have a mass median aerodynamic diameter of about 20 micrometres or greater, or about 50 micrometres or greater, or in a range from about 50 to about 200 micrometres, or from about 50 to about 150 micrometres. The mass median aerodynamic diameter is preferably measured with a cascade impactor.

The dry powder may have a mean diameter of about 60 micrometres or less, or in a range from about 1 micrometres to about 40 micrometres, or in a range from about 1.5 micrometres to about 25 micrometres. The mean diameter refers to the mean diameter per mass and is preferably measured by laser diffraction, laser diffusion or an electronic microscope.

Nicotine in the powder system or nicotine particles may be a pharmaceutically acceptable free-base nicotine, or nicotine salt or nicotine salt hydrate. Useful nicotine salts or nicotine salt hydrates include nicotine pyruvate, nicotine citrate, nicotine aspartate, nicotine lactate, nicotine bitartrate, nicotine salicylate, nicotine fumarate, nicotine mono-pyruvate, nicotine glutamate or nicotine hydrochloride, for example. The compound combining with nicotine to form the salt or salt hydrate may be chosen based on its expected pharmacological effect.

The nicotine particles preferably include an amino acid. Preferably the amino acid may be leucine such as L-leucine. Providing an amino acid such as L-leucine with the particles comprising nicotine, may reduce adhesion forces of the particles comprising nicotine and may reduce attraction between nicotine particles and thus reduce agglomeration of nicotine particles. Similarly, adhesion forces to particles comprising flavour may also be reduced thus agglomeration of nicotine particles with flavour particles is also reduced. The powder system described herein thus may be a free-flowing material and possess a stable relative particle size of each powder component even when the nicotine particles and the flavour particles are combined.

Preferably, the nicotine is a surface modified nicotine salt where the nicotine salt particle comprises a coated or composite particle. A preferred coating or composite material may be L-leucine. One particularly useful nicotine particle may be nicotine bitartrate with L-leucine.

The powder system may include a population of flavour particles. The flavour particles may have any useful size distribution for inhalation delivery selectively into the mouth or buccal cavity of a user.

The powder system may have at least about 40%, or at least about 60%, or at least about 80%, by weight of the population of flavour particles of the powder system comprised in particles having a particle size of about 20 micrometres or greater. The powder system may have at least about 40% or at least about 60%, or at least about 80%, by weight of the population of flavour particles of the powder system comprised in particles having a particle size of about 50 micrometres or greater. The powder system may have at least about 40% or at least about 60%, or at least about 80%, by weight of the population of flavour particles of the powder system comprised in particles having a particle size in a range from about 50 micrometre to about 150 micrometres.

The particles comprising flavour may include a compound to reduce adhesion forces or surface energy and resulting agglomeration. The flavour particle may be surface modified with an adhesion reducing compound to form a coated flavour particle. One preferred adhesion reducing compound may be magnesium stearate. Providing an adhesion reducing compound such as magnesium stearate with the flavour particle, especially coating the flavour particle, may reduce adhesion forces of the particles comprising flavour and may reduce attraction between flavour particles and thus reduce agglomeration of flavour particles. Thus, agglomeration of flavour particles with nicotine particles may also be reduced. The powder system described herein thus may possess a stable relative particle size of the particles comprising nicotine and the particles comprising flavour even when the nicotine particles and the flavour particles are combined. The powder system preferably may be free flowing.

Conventional formulations for dry powder inhalation contain carrier particles that serve to increase the fluidization of the active particles since the active particles may be too small to be influenced by simple air flow though the inhaler. The powder system may comprise carrier particles. These carrier particles may be a saccharide such as lactose or mannitol that may have a particle size greater than about 50 micrometres. The carrier particles may be utilized to improve dose uniformity by acting as a diluent or bulking agent in a formulation.

The powder system utilized with the nicotine powder delivery system described herein may be carrier-free or substantially free of a saccharide such as lactose or mannitol. Being carrier-free or substantially free of a saccharide such as lactose or mannitol may allow the nicotine and to be inhaled and delivered to the user's lungs at inhalation or air flow rates that are similar to typical smoking regime inhalation or air flow rates.

The nicotine particles and a flavour may be combined in a single capsule. As described above, the nicotine particles and a flavour may each have reduced adhesion forces that result in a stable particle formulation where the particle size of each component does not substantially change when combined. Alternatively, the powder system includes nicotine particles contained within a single capsule and the flavour particles contained within a second capsule.

The nicotine particles and flavour particles may be combined in any useful relative amount so that the flavour particles are detected by the user when consumed with the nicotine particles. Preferably the nicotine particles and flavour particles form at least about 90%wt or at least about 95%wt or at least about 99%wt or 100% wt of the total weight of the powder system.

The inhaler and inhaler system may be less complex and have a simplified air flow path as compared to conventional dry powder inhalers. Advantageously, rotation of the capsule within the inhaler article aerosolizes the nicotine particles or powder system and may assist in maintaining a free-flowing powder. Thus, the inhaler article may not require the elevated inhalation rates typically utilized by conventional inhalers to deliver the nicotine particles described above deep into the lungs.

The inhaler article may use a flow rate of less than about 5 L/min or less than about 3 L/min or less than about 2 L/min or about 1.6 L/min. Preferably, the flow rate may be in a range from about 1 L/min to about 3 L/min or from about 1.5 L/min to about 2.5 L/min. Preferably, the inhalation rate or flow rate may be similar to that of Health Canada smoking regime, that is, about 1.6 L/min.

The inhaler system may be used by a consumer like smoking a conventional cigarette or vaping an electronic cigarette. Such smoking or vaping may be characterized by two steps: a first step during which a small volume containing the full amount of nicotine desired by the consumer is drawn into the mouth cavity, followed by a second step during which this small volume comprising the aerosol comprising the desired amount of nicotine is further diluted by fresh air and drawn deeper into the lungs. Both steps are controlled by the consumer. During the first inhalation step the consumer may determine the amount of nicotine to be inhaled. During the second step, the consumer may determine the volume for diluting the first volume to be drawn deeper into the lungs, maximizing the concentration of active agent delivered to the airway epithelial surface. This smoking mechanism is sometimes called "puff-inhale-exhale".

The dry powder utilized with the dry powder inhaler of the disclosure may eliminate or substantially reduce any exhalation of pharmaceutically active particles during the "exhale" phase. Preferably nearly all, or at least about 99% or at least about 95% or at least 90% of the pharmaceutically active particle has a particle size that is delivered to the lungs but are not small enough to be exhaled by tidal breathing. This pharmaceutically active particle size may be in a range from about 0.75 micrometres to about 5 micrometres, or from 0.8 micrometres to about 3 micrometres, or from 0.8 micrometres to about 2 micrometres.

As noted above, the inhaler article comprises a hollow tubular element, which comprises: a peripheral portion defining a hollow inner region of the hollow tubular element; and a support element formed from a sheet and extending from a first point at the peripheral portion across the hollow inner region to a second point at the peripheral portion.

The peripheral portion may be referred to as a peripheral portion of material. The peripheral portion may be formed from a sheet. The peripheral portion and the support element may be integrally formed from a sheet. In other words, the peripheral portion and the support element may be formed from the same sheet. The peripheral portion and the support element may be formed from separate sheets.

The peripheral portion may comprise a tube. The peripheral portion may be formed from a tube. The tube may be distinct from the sheet which forms the support element. The tube may be formed from a sheet that is the same as or distinct from the sheet which forms the support element. For example, the peripheral portion may comprise a tube which is distinct from the sheet that forms the support element; a first end of the sheet that forms the support element may be in contact with the tube up to a first point at the peripheral portion, where it deflects away from the tube and into the hollow inner region; a second end of the sheet that forms the support element may be in contact with the tube up to a second point at the peripheral portion, where it deflects away from the tube and into the hollow inner region; the portion of the sheet between the first point at the peripheral portion and the second point at the peripheral portion may form a support element which extends from the first point at the peripheral portion across the hollow inner region to the second point at the peripheral portion. In this instance, the peripheral portion comprises the portion of the sheet extending from the first end of the sheet to the first point at the peripheral portion, and the portion of the sheet extending from the second point at the peripheral portion to the second end of the sheet.

Where the peripheral portion comprises a tube, the sheet forming the support element may be attached to the tube by an adhesive at points where the sheet is in contact with the tube.

The peripheral portion may form an outer surface of the hollow tubular element. Where the peripheral portion is formed from a sheet, preferably the portion of the sheet forming the peripheral portion forms an outer surface of the hollow tubular element. Substantially the entirety of the portion of the sheet forming the peripheral portion may form an outer surface of the hollow tubular element. An outer surface of the hollow tubular element may be curved.

The support element may extend along part of the length of the hollow tubular element. Preferably, the support element extends from the upstream end of the hollow tubular element. This means that the support element may be at the end of the hollow tubular element closest to the capsule. As such, the support element may be better able to prevent or restrict movement of the capsule, for example when the capsule is being pierced. Preferably, the support element extends to the downstream end of the hollow tubular element. The support element may extend along between about 10 percent and about 100 percent of the length of the hollow tubular element, preferably along between about 25 percent and about 100 percent of the length of the hollow tubular element, more preferably along between about 50 and about 100 percent of the length of the hollow tubular element. Most preferably, the support element extends along substantially the entire length of the hollow tubular element. As such, the support element may have a length equal to about the length of the hollow tubular element. This may provide the hollow tubular element with additional mechanical strength and stiffness along the entire length of the hollow tubular element.

The support element may depend from the peripheral portion along a first fold line of the sheet which forms the support element, wherein the first fold line resides at the first point at the peripheral portion. Advantageously, this may simplify manufacturing of the hollow tubular element and may provide a suitable support barrier for one or more components disposed upstream of the hollow tubular element, such as the capsule.

The sheet which forms the support element may also form part of the peripheral portion. For example, a portion of the sheet adjacent to the first fold line and on the other side of the first fold line from the support element, may form part of the peripheral portion. This portion of the sheet may be attached to the remainder of the peripheral portion by an adhesive. The use of an adhesive can help to improve the mechanical strength of the hollow tubular element in one or both of the longitudinal direction and the transverse direction. As such, this can help to improve the hollow tubular element's ability to provide a support barrier and its resistance to collapse or deformation. The portion of the sheet adjacent to the first fold line, and on the other side of the first fold line from the support element, may form the entirety of the peripheral portion.

The first fold line may extend along part of the length of the hollow tubular element. In this case, the support element also extends along part of the length of the hollow tubular element. Preferably, the first fold line extends from the upstream end of the hollow tubular element. Preferably, the first fold line extends to the downstream end of the hollow tubular element. The first fold line may extend along about 10 percent or more of the length of the hollow tubular element, preferably along about 25 percent or more of the length of the hollow tubular element, more preferably along about 50 percent or more of the length of the hollow tubular element. Most preferably, the first fold line extends along substantially the entire length of the hollow tubular element.

The first fold line may be parallel to the longitudinal axis of the hollow tubular element. The first fold line may be non-parallel to the longitudinal axis of the hollow tubular element. The first fold line may be designed to be non-parallel to the longitudinal axis of the hollow tubular element in such a way that the internal projection induces a swirling air flow pattern within the hollow inner region of the hollow tubular element.

Where a sheet comprises a fold line, the sheet may be deflected by an angle of greater than about 45 degrees about the fold line, greater than about 60 degrees about the fold line, greater than about 75 degrees about the fold line, or greater than about 90 degrees about the fold line.

The fold line may be a crease line. The sheet may comprise a score line aligned with the fold line to assist with folding of the sheet.

The first fold line may be the only fold line along which the support element depends from the peripheral portion.

The support element may comprise an end of the sheet. The end of the sheet may be in contact with the peripheral portion at the second point at the peripheral portion. The end of the sheet may be attached to the peripheral portion at the second point at the peripheral portion by an adhesive.

Preferably, the support element depends from the peripheral portion along a second fold line of the sheet, wherein the second fold line resides at the second point at the peripheral portion. This may provide the hollow tubular with sufficient mechanical strength and stiffness in one or both of the longitudinal direction and the transverse direction to prevent or restrict movement of one or more components disposed upstream of the hollow tubular element, such as the capsule, without significant deformation of the hollow tubular element during use of the inhaler article, for example during interaction of the inhaler article with a holder for receiving the inhaler article. The second fold line may extend along part of the length of the hollow tubular element. The second fold line may extend along about 10 percent or more of the length of the hollow tubular element, preferably along about 25 percent or more of the length of the hollow tubular element, more preferably along about 50 percent or more of the length of the hollow tubular element. Most preferably, the second fold line extends along substantially the entire length of the hollow tubular element.

Preferably, the first fold line and the second fold line extend along the length of the hollow tubular element by about the same amount.

The first fold line and the second fold line may be parallel to each other. The first fold line and the second fold line may be non-parallel to each other.

Preferably, the first point at the peripheral portion and the second point at the peripheral portion have the same longitudinal position. That is, the first point at the peripheral portion and the second point at the peripheral portion are preferably in the same transverse cross-sectional plane.

The first point at the peripheral portion and the second point at the peripheral portion may be spaced apart from each other. The first point at the peripheral portion and the second point at the peripheral portion may be spaced apart from each other by about 0.05 millimetres or more, preferably about 0.3 millimetres or more, more preferably about 0.5 millimetre or more.

The first point at the peripheral portion and the second point at the peripheral portion may be spaced apart from each other by about 3 millimetres or less, preferably about 2.5 millimetres or less, more preferably about 2 millimetres or less.

The first point at the peripheral portion and the second point at the peripheral portion may be spaced apart from each other by between about 0.05 millimetres and about 3 millimetres, preferably between about 0.3 millimetres and about 2.5 millimetres, more preferably between about 0.5 millimetres and about 2 millimetres.

The first point at the peripheral portion and the second point at the peripheral portion may be spaced apart from each other around the circumference of the hollow tubular element by about 0.2 percent or more of the circumference of the hollow tubular element, preferably about 2 percent or more of the circumference of the hollow tubular element, more preferably about 3 percent or more of the circumference of the hollow tubular element.

The first point at the peripheral portion and the second point at the peripheral portion may be spaced apart from each other around the circumference of the hollow tubular element by about 12 percent or less of the circumference of the hollow tubular element, preferably about 10 percent or less of the circumference of the hollow tubular element, more preferably about 8 percent or less of the circumference of the hollow tubular element

The first point at the peripheral portion and the second point at the peripheral portion may be spaced apart from each other around the circumference of the hollow tubular element by between about 0.2 percent and about 12 percent of the circumference of the hollow tubular element, preferably between about 2 percent and about 10 percent of the circumference of the hollow tubular element, more preferably between about 3 percent and about 8 percent of the circumference of the hollow tubular element.

The first point at the peripheral portion and the second point at the peripheral portion may be spaced apart from each other around the circumference of the hollow tubular element by about half of the circumference of the hollow tubular element. That is, the first point at the peripheral portion and the second point at the peripheral portion may be about diametrically opposed to each other.

The first point at the peripheral portion and the second point at the peripheral portion may be spaced apart from each other around the circumference of the hollow tubular element by between about 5 percent and about 50 percent of the circumference of the hollow tubular element, preferably between 10 percent and about 40 percent of the circumference of the hollow tubular element, more preferably between about 15 percent and about 30 percent of the circumference of the hollow tubular element.

The first point at the peripheral portion and the second point at the peripheral portion may be adjacent to each other. The first point at the peripheral portion and the second point at the peripheral portion may be spaced apart from each other by about zero millimetres. The first point at the peripheral portion and the second point at the peripheral portion may be in contact with each other. The first point at the peripheral portion and the second point at the peripheral portion may be attached to each other by an adhesive. The use of an adhesive can help to improve the mechanical strength of the hollow tubular element in one or both of the longitudinal direction and the transverse direction. As such, this can help to improve the hollow tubular element's resistance to collapse or deformation.

The support element may be in contact with the peripheral portion at a further point at the peripheral portion other than the first point at the peripheral portion and other than the second point at the peripheral portion. Where the support element is in contact with the peripheral portion, the support element may be attached to that point at the peripheral portion by an adhesive.

The support element may comprise a tip, the tip being positioned within the hollow inner region. The tip may be spaced apart from the peripheral portion. The tip may be spaced apart from the peripheral portion by about 0.6 millimetres or more, preferably about 1.5 millimetres or more, more preferably about 2 millimetres or more, or about 3 millimetres or more.

The tip may be spaced apart from the radial centre of the hollow tubular element by about 0.2 millimetres or more, preferably about 0.5 millimetres or more, more preferably about 1 millimetre or more.

The tip may be spaced apart from the radial centre of the hollow tubular element by about 3 millimetres or less, preferably about 2.5 millimetres or less, more preferably about 2 millimetres or less.

The tip may be spaced apart from the radial centre of the hollow tubular element by between about 0.2 millimetres and about 3 millimetres, preferably between about 0.5 millimetres and about 2.5 millimetres, more preferably about 1 millimetre and about 2 millimetres.

The tip may be spaced apart from the radial centre of the hollow tubular element by about 1.5 millimetres.

The tip may reside at a point which is adjacent to a point at the peripheral portion. The tip may be in contact with the peripheral portion. The tip may reside at the radial centre of the hollow tubular element.

The tip may be positioned about equidistant from the first point at the peripheral portion and the second point at the peripheral portion.

As used herein, the term "radial centre" is used to refer to the centre of a transverse cross section of the hollow tubular element.

The tip may be pointed. For example, the support element may have a substantially triangular cross section.

The tip may be rounded. For example, the support element may have a substantially parabolic cross section.

The tip may be flat. For example, the support element may have a substantially trapezoidal cross section.

The support element may comprise a third fold line of the sheet. That is, the sheet forming the support element may comprise a third fold line between the first point at the peripheral portion and the second point at the peripheral portion. The support element may comprise a third fold line of the sheet between the first fold line and the second fold line. This may further strengthen the hollow tubular element in one or both of the longitudinal direction and the transverse direction to enable the hollow tubular element to withstand larger forces being applied to it in one or both of the longitudinal direction and the transverse direction before deforming substantially. As such, this may improve the hollow tubular element's ability to prevent or restrict movement of one or more components disposed upstream of the hollow tubular element, such as the capsule.

The third fold line may reside at or adjacent to the peripheral portion. The third fold line may reside at or adjacent to the radical centre of the hollow tubular element.

The third fold line may define the tip of the support element.

The third fold line may be positioned about equidistant from the first fold line and the second fold line. The third fold line may be positioned closer to the first fold line than the second fold line.

Preferably, there is about the same amount of material of the sheet between the first fold line and the third fold line as there is between the second fold line and the third fold line. There may be less material of the sheet between the first fold line and the third fold line than there is between the second fold line and the third fold line.

A surface of the support element along the longitudinal direction may be substantially planar. As such, a cross section of the hollow tubular element may comprise a straight line corresponding to the substantially planar surface of the support element along the longitudinal direction. The substantially planar surface may extend from the first point at the peripheral portion. The substantially planar surface may extend to the second point at the peripheral portion. The substantially planar surface may extend from the first point at the peripheral portion to the second point at the peripheral portion. Where there is a first fold line of the sheet, the substantially planar surface may extend from the first fold line. Where there is a second fold line of the sheet, the substantially planar surface may extend to the second fold line. Where there is both a first fold line of the sheet and a second fold line of the sheet, the substantially planar surface may extend from the first fold line to the second fold line. Where there is both a first fold line of the sheet and a third fold line of the sheet, the substantially planar surface may extend from the first fold line to third fold line. Where there is both a second fold line of the sheet and a third fold line of the sheet, the substantially planar surface may extend from the second fold line to the third fold line.

The support element may comprise a substantially straight portion, when viewed from the upstream end of the hollow tubular element. The substantially straight portion may extend from the first point at the peripheral portion, when viewed from the upstream end of the hollow tubular element. The substantially straight portion may extend to the second point at the peripheral portion, when viewed from the upstream end of the hollow tubular element. The substantially straight portion may extend from the first point at the peripheral portion to the second point at the peripheral portion, when viewed from the upstream end of the hollow tubular element. In particular, where there is a first fold line of the sheet, the substantially straight portion may extend from the first fold line of the sheet, when viewed from the upstream end of the hollow tubular element. Where there is a second fold line of the sheet, the substantially straight portion may extend to the second fold line, when viewed from the upstream end of the hollow tubular element. Where there is both a first fold line and a second fold line of the sheet, the substantially planar surface may extend from the first fold line to the second fold line, when viewed from the upstream end of the hollow tubular element. Where there is both a first fold line of the sheet and a third fold line of the sheet, the substantially straight portion may extend from the first fold line to third fold line, when viewed from the upstream end of the hollow tubular element. Where there is both a second fold line of the sheet and a third fold line of the sheet, the substantially straight portion may extend from the second fold line to the third fold line, when viewed from the upstream end of the hollow tubular element.

Where there is a both a first fold line and a third fold line, the first fold line and the third fold line may define a first side wall of the support element. That is, the first side wall may extend from the first fold line to the third fold line and there are no fold lines therebetween. The first side wall may be substantially straight. The first side wall may be curved.

The first side wall may be wholly enclosed by the peripheral portion of the hollow tubular element and therefore, does not form an outer surface of the hollow tubular element.

Where there is both a second fold line and a third fold line, the second fold line and the third fold line may define a second side wall of the support element. That is, the second side wall may extend from the second fold line to the third fold line and there are no fold lines therebetween. The second side wall may be substantially straight. The second side wall may be curved.

The second side wall may be wholly enclosed by the peripheral portion of the hollow tubular element and therefore, does not form an outer surface of the hollow tubular element.

The first side wall of the support element may form an outer surface of the hollow tubular element. The second side wall of the support element may form an outer surface of the hollow tubular element. For example, the hollow tubular element may comprise a peripheral portion and a support element formed integrally from the same sheet; wherein substantially the entirety of the peripheral portion and substantially the entirety of the support element are formed from a single layer of the sheet (excluding a seam); wherein the support element depends from the peripheral portion along both a first fold line of the sheet and a second fold line of the sheet; wherein the support element comprises a third fold line residing within the hollow inner region of the hollow tubular element, the first fold line and the third fold line defining a substantially straight first side wall of the support element, the second fold line and the third fold line defining a substantially straight second side wall of the support element; and wherein the first side wall and the second side wall from an angle of, for example, 30 degrees about the third fold line. In this example the first side wall forms an outer surface of the hollow tubular element, and the second side wall forms an outer surface of the hollow tubular element.

The outer surface of the hollow tubular element may be formed from the peripheral portion, the first side wall of the support element and the second side wall of the support element.

Where the first side wall is substantially straight and the second side wall is substantially straight, the first side wall and the second side wall may define an angle of about 5 degrees or more therebetween. That is, the angle between the first side wall and the second side wall may be about 5 degrees or more. In other words, the angle about the third fold line may be about 5 degrees or more. Preferably, the angle between the first side wall and the second side wall at the third fold line is about 10 degrees or more, more preferably about 15 degrees or more, even more preferably about 20 degrees or more.

Where the first side wall is substantially straight and the second side wall is substantially straight, the angle between the first side wall and the second side wall may be about 50 degrees or less, preferably the angle between the first side wall and the second side wall at the third fold line is about 45 degrees or less, more preferably about 40 degrees or less, even more preferably about 35 degrees or less.

Where the first side wall is substantially straight and the second side wall is substantially straight, the angle between the first side wall and the second side wall may be between about 5 degrees and about 50 degrees, preferably between about 10 degrees and about 45 degrees, more preferably between about 15 degrees and about 40 degrees, even more preferably between about 20 degrees and about 35 degrees.

A surface of the first side wall and a surface of the second side wall may be in contact with each other. A surface of the first side wall and a surface of the second side wall may be attached to each other by an adhesive. Substantially the entire outer surface of the first side wall and substantially the entire outer surface of the second side wall may be in contact with each other. Substantially the entire outer surface of the first side wall and substantially the entire outer surface of the second side wall may be attached to each other by an adhesive. The use of an adhesive can help to improve the mechanical strength of the hollow tubular element in one or both of the longitudinal direction and the transverse direction. As such, this can help to improve the hollow tubular element's resistance to collapse or deformation and the hollow tubular element's ability to prevent or restrict movement of one or more components disposed upstream of the hollow tubular element, such as the capsule. Where the first side wall is substantially straight and the second side wall is substantially straight, the angle formed between the first side wall and the second side wall may be approximately zero degrees.

A cross section of the support element may comprise a curved portion. The support element may comprise a curved portion, when viewed from the upstream end of the hollow tubular element. The support element may comprise a substantially s-shaped cross section. The support element may be substantially s-shaped, when viewed from the upstream end of the hollow tubular element. The support element may comprise a substantially omega-shaped cross section. The support element may be substantially omega-shaped, when viewed from the upstream end of the hollow tubular element. The support element may comprise a substantially c-shaped cross section. The support element may be substantially c-shaped, when viewed from the upstream end of the hollow tubular element.

The support element may have a wave profile as viewed from the upstream end of the hollow tubular element. The support element may comprise a plurality of peaks and troughs, when viewed from the upstream end of the hollow tubular element. The support element may be substantially sinusoidal, when viewed from the upstream end of the hollow tubular element. The support element may have a substantially triangular wave profile as viewed form the upstream end of the hollow tubular element. For example, the support element may be substantially w-shaped as viewed from the upstream end of the hollow tubular element.

The hollow tubular element may comprise at least one longitudinal plane of symmetry. The hollow tubular element may be radially symmetric. This may simplify assembling of the inhaler article, since the orientation in which the hollow tubular element is disposed in the inhaler article may be less important. In addition, this may also mean that the hollow tubular element is able to distribute load more evenly to be able to withstand increased forces being applied to it.

Preferably, the cross-sectional area of the hollow tubular element is substantially constant along the entire length of the hollow tubular element. This may be such that the resistance to draw of the inhaler article is also constant along the entire length of the hollow tubular element.

Preferably, the hollow tubular element has a substantially constant cross section along the entire length of the hollow tubular element. That is, the cross section of the hollow tubular element does not change substantially along the entire length of the hollow tubular element. This may simplify manufacturing of the hollow tubular element. Alternatively, the cross section of the hollow tubular element may vary along the length of the hollow tubular element. For example, the support element may have a cross section that varies along the length of the hollow tubular element. For instance, the support element may not extend along the entire length of the hollow tubular element.

The support element may divide the hollow inner region of the hollow tubular element into a plurality of channels. The number of channels may be selected based on a desired nucleation of aerosol particles and a desired resistance to draw of the inhaler article. The support element may divide the cavity of the hollow tubular element into two channels. The support element may divide the cavity of the hollow tubular element into three channels. The support element may divide the cavity of the hollow tubular element into four channels.

The support element may divide the cavity of the hollow tubular element into between two channels and four channels. The support element may divide the cavity of the hollow tubular element into at least three channels.

The support element may extend through the radial centre of the hollow tubular element.

The support element may be spaced apart from the radial centre of the hollow tubular element by a distance of about 5 percent or more of the radius of the hollow tubular element, preferably about 10 percent or more of the radius of the hollow tubular element, more preferably about 15 percent or more of the radius of the hollow tubular element.

The support element may be spaced apart from the radial centre of the hollow tubular element by a distance of about 90 percent or less of the radius of the hollow tubular element, preferably about 80 percent or less of the radius of the hollow tubular element from the radial centre of the hollow tubular element, more preferably about 70 percent or less of the radius of the hollow tubular element from the radial centre of the hollow tubular element.

The support element may be spaced apart from the radial centre of the hollow tubular element by a distance of between about 5 percent and about 90 percent of the radius of the hollow tubular element, preferably between about 10 percent and about 80 percent of the radius of the hollow tubular element, more preferably between about 15 percent and about 70 percent of the radius of the hollow tubular element.

The support element may be spaced apart from the radial centre of the hollow tubular element by a distance of about 0.2 millimetres or more, preferably about 0.5 millimetres or more, more preferably about 1 millimetre or more from the radial centre of the hollow tubular element.

The support element may be spaced apart from the radial centre of the hollow tubular element by a distance of about 3 millimetres or less, preferably about 2.5 millimetres or less, more preferably about 2 millimetres or less, or about 1 millimetre or less.

The support element may be spaced apart from the radial centre of the hollow tubular element by a distance of between about 0.2 millimetres and about 3 millimetres, preferably between about 0.5 millimetres and about 2.5 millimetres, more preferably between about 1 millimetre and about 2 millimetres, or between about 0.5 millimetres and about 1 millimetre.

Where the support element comprises a tip, the support element may have a depth of about 0.6 millimetres or more, preferably about 1 millimetre or more, more preferably about 1.5 millimetres or more.

Where the support element comprises a tip, the support element may have a depth of about 3 millimetres or less, preferably about 2.7 millimetres or less, more preferably about 2.5 millimetres or less.

Where the support element comprises a tip, the support element may have a depth of between about 0.6 millimetres and about 3 millimetres, preferably between about 1 millimetre and about 2.7 millimetres, more preferably between about 1.5 millimetres and about 2.5 millimetres. Where the support element comprises a tip, the support element may have a depth of between about 2 millimetres and about 3 millimetres.

Where the support element comprises a tip, the support element may have a depth of about 2 millimetres. Where the support element comprises a tip, the support element may have a depth equal to about the inner radius of the hollow tubular element.

As used herein, the term "depth" denotes the distance between the first point at the peripheral portion and the tip of the support element.

The support element may be the only support element of the hollow tubular element. That is, the hollow tubular element may comprise a single support element. Alternatively, the support element may be a first support element and the hollow tubular element may comprise one or more additional support elements. Each of the one or more additional support elements may be formed from a sheet. The one or more additional support elements may be formed from separate sheets. Preferably, the one or more additional support elements are formed from the same sheet as the first support element. Each of the one or more additional support elements may extend from a respective first point at the peripheral portion across the hollow inner region to a respective second point at the peripheral portion.

The one or more additional support elements may depend from the peripheral portion along a respective first fold line of the sheet, wherein the respective first fold line resides at the respective first point at the peripheral portion. The one or more additional support elements may depend from the peripheral portion along a respective second fold line of the sheet, wherein the respective second fold line resides at the respective second point at the peripheral portion.

The hollow tubular element may comprise between two and six support elements. Preferably, the hollow tubular element comprises three support elements. Three support elements can help to improve the hollow tubular element's resistance to collapse or deformation and the hollow tubular element's ability to prevent or restrict movement of one or more components disposed upstream of the hollow tubular element, such as the capsule.

Each of the support elements may be identical to one another. This may simplify manufacturing of the hollow tubular element. Alternatively, one of the support elements may be different to another support element. For example, the first support element may be larger in size than the second support element.

Each of the support elements may have any combination of the features described above in respect of the support element, that is, the first support element.

Each of the support elements may be about equally spaced around the peripheral portion of the hollow tubular element. This means that the separation between the first point at the peripheral portion from which one of the support element extends and the first point at the peripheral portion from which the next support element extends is about the same around the peripheral portion of the hollow tubular element.

Where the support elements are identical to one another and are equally spaced around the peripheral portion of the hollow tubular element, the hollow tubular element may comprise radial symmetry. This may simplify assembling of the inhaler article, since the orientation in which the hollow tubular element is disposed in the inhaler article may be less important. In addition, this may also mean that the hollow tubular element is able to distribute load more evenly to be able to withstand increased forces being applied to it.

The hollow tubular element preferably has an outer diameter that is approximately equal to the outer diameter of the inhaler article.

The hollow tubular element may have an outer diameter of about 5 millimetres or more, preferably about 6 millimetres or more, more preferably about 7 millimetres or more.

The hollow tubular element may have an outer diameter of about 12 millimetres or less, preferably about 10 millimetres or less, more preferably about 8 millimetres or less.

The hollow tubular element may have an outer diameter of between about 5 millimetres and about 12 millimetres, preferably between about 6 millimetres and about 10 millimetres, more preferably, between about 7 millimetres and about 8 millimetres.

The hollow tubular element may have an outer diameter of about 7.2 millimetres.

The hollow tubular element may have an inner diameter of about 4.5 millimetres or more, preferably about 5.5 millimetres or more, more preferably about 6.5 millimetres or more.

The hollow tubular element may have an inner diameter of about 11.5 millimetres or less, preferably about 9.5 millimetres or less, more preferably about 7.5 millimetres or less.

The hollow tubular element may have an inner diameter of between about 4.5 millimetres and about 11.5 millimetres, preferably between about 5.5 millimetres and about 9.5 millimetres, more preferably between about 6.5 millimetres and about 7.5 millimetres.

The hollow tubular element may have a total internal surface area of about 25 millimetres squared per millimetre length or more, preferably about 28 millimetres squared per millimetre length or more, more preferably about 30 millimetres squared per millimetre length or more, or about 35 millimetres squared per millimetre length or more.

The hollow tubular element may have a total internal surface area of about 70 millimetres squared per millimetre length or less, preferably about 60 millimetres squared per millimetre length or less, more preferably about 50 millimetres squared per millimetre length or less, or about 40 millimetres squared per millimetre length or less.

The hollow tubular element may have a total internal surface area of between about 25 millimetres squared per millimetre length and about 70 millimetres squared per millimetre length, preferably between about 28 millimetres squared per millimetre length and about 60 millimetres squared per millimetre length, more preferably between about 30 millimetres squared per millimetre length and about 50 millimetres squared per millimetre length, or between about 30 millimetres squared per millimetre length and about 40 millimetres squared per millimetre length. The hollow tubular element may have a total internal surface area of between about 35 millimetres squared per millimetre length and about 70 millimetres squared per millimetre length, preferably between about 40 millimetres squared per millimetre length and about 70 millimetres squared per millimetre length, more preferably between about 50 millimetres squared per millimetre length and about 70 millimetres squared per millimetre length, or between about 60 millimetres squared per millimetre length and about 70 millimetres squared per millimetre length.

Preferably, the hollow tubular element provides an unrestricted flow channel. This means that the hollow tubular segment preferably provides a negligible level of resistance to draw (RTD). The term "negligible level of RTD" is used to describe an RTD of less than 1 mm H2O per 10 millimetres of length of the hollow tubular element, preferably less than 0.4 mm H2O per 10 millimetres of length of the hollow tubular element, more preferably less than 0.1 mm H2O per 10 millimetres of length of the hollow tubular element. The flow channel should therefore be free from any components that would obstruct the flow of air in a longitudinal direction. Preferably, the flow channel is substantially empty.

The hollow tubular element may have a porosity of about 90 percent or more in the longitudinal direction.

As used herein, the porosity of the hollow tubular element in the longitudinal direction is defined by the ratio of the cross-sectional area of material forming the hollow tubular element and the internal cross-sectional area of the inhaler article at the position of the hollow tubular element.

The porosity in the longitudinal direction of the hollow tubular element may advantageously be selected in order to provide a desirable overall resistance to draw of the inhaler article.

The porosity in the longitudinal direction of the hollow tubular element may be substantially constant along the entire length of the hollow tubular element. For example, the cross-sectional area of material forming the hollow tubular element may be substantially constant along the entire length of the hollow tubular element and the inhaler article may also have a substantially constant internal cross-sectional area along the entire length of the hollow tubular element. The hollow tubular element may have a substantially constant cross section along the entire length of the hollow tubular element such that the cross-sectional area of material forming the hollow tubular element is substantially constant along the entire length of the hollow tubular element. The hollow tubular element may also have a cross section that varies along the length of the hollow tubular element and a substantially constant cross-sectional area of material forming the hollow tubular element along the entire length of the hollow tubular element.

The porosity in the longitudinal direction of the hollow tubular element may vary along the length of the hollow tubular element. For example, this may be the case where the hollow tubular element does not have a constant cross section along the entire length of the hollow tubular element such that the cross-sectional area of material forming the hollow tubular element varies along the length of the hollow tubular element.

The sheet forming one or both of the support element and the peripheral portion may be formed from paper, any other paper-based material, any other cellulose-based material, a bioplastic-based material, or a metal. For example, the sheet may be formed from one or more of paper, paperboard, cardboard, reconstituted tobacco paper, cellophane and aluminium.

Preferably the sheet is formed from a biodegradable material.

Most preferably, the sheet is formed from a paper-based material, such as paper, paperboard or cardboard. The paper-based material may be bleached or unbleached. Paper-based materials may be one or more of light, cheap and biodegradable. Where one or both of the support element and the peripheral portion is formed from a paper sheet, the hollow tubular element is able to prevent or restrict movement of one or more components disposed upstream of the hollow tubular element, such as the capsule, whilst exhibiting sufficient mechanical strength and stiffness to withstand significant deformation during interaction of the inhaler article with holder for receiving the inhaler article. The interaction may involve insertion of the inhaler article into the holder. The material properties of a paper sheet may be such that individual hollow tubular elements comprising a peripheral portion and a support element, wherein one or both of the peripheral portion and the support element is formed from a paper sheet, may be cut from a continuous rod of hollow tubular element. This may simplify manufacturing of the hollow tubular element.

The sheet forming one or both of the peripheral portion and the support element may have a basis weight of about 15 grams per square metre or more, preferably about 25 grams per square metre or more, more preferably about 35 grams per square metre or more, or about 45 grams per square metre or more. A sheet with such basis weight may avoid one or both of crack formation and breakage during one or both of bending and folding of the sheet. As such, the sheet may retain its structural integrity when bent or folded to form the support element. This may improve the hollow tubular element's resistance to collapse or deformation and the hollow tubular element's ability to prevent or restrict movement of one or both of at least part of the aerosol-forming substrate and at least part of the susceptor element.

The sheet forming one or both of the peripheral portion and the support element may have a basis weight of about 150 grams per square metre or less, preferably about 130 grams per square metre or less, more preferably about 110 grams per square metre or less, or about 80 grams per square metre or less, or about 50 grams per square metre or less. Providing a sheet with such basis weight may advantageously ensure that the hollow tubular element has a desired porosity in the longitudinal direction. This may be such that the hollow tubular element has a desired resistance to draw. In addition, providing a sheet with such basis weight may advantageously make the hollow tubular element easier to manufacture, for example, by making the sheet easier to at least one of roll, bend and fold the sheet.

The sheet may have a basis weight of between about 15 grams per square metre and about 150 grams per square metre, between about 20 grams per square metre and about 130 grams per square metre, between about 60 grams per square metre and about 100 grams per square metre, between about 70 grams per square metre and about 80 grams per square metre.

Preferably, the sheet has a basis weight of between about 45 grams per square metre and about 110 grams per square metre. The sheet may have a basis weight of about 45 grams per square metre. The sheet may have a basis weight of about 60 grams per square metre. Preferably, the sheet has a basis weight of about 78 grams per square metre. Preferably, the sheet has a basis weight of 110 grams per square metre.

The sheet forming one or both of the peripheral portion and the support element may have a thickness of about 15 micrometres or more, about 30 micrometres or more, about 45 micrometres or more, about 100 micrometres or more. A sheet with such thickness may avoid one or both of crack formation and breakage during one or both of bending and folding of the sheet. As such, the sheet may retain its structural integrity when bent or folded to form the support element. This may improve the hollow tubular element's resistance to collapse or deformation and the hollow tubular element's ability to prevent or restrict movement of one or both of at least part of the aerosol-forming substrate and at least part of the susceptor element.

The sheet forming one or both of the peripheral portion and the support element may have a thickness of about 150 micrometres or less, preferably about 140 micrometres or less, more preferably about 130 micrometres or less. Providing a sheet with such thickness may advantageously ensure that the hollow tubular element has a desired porosity in the longitudinal direction. This may be such that the hollow tubular element has a desired resistance to draw. In addition, providing a sheet with such basis weight may advantageously make the hollow tubular element easier to manufacture, for example, by making the sheet easier to at least one of roll, bend and fold the sheet.

The sheet may have a thickness of between about 15 micrometres and about 150 micrometres, preferably between about 30 micrometres and about 140 micrometres, more preferably between about 100 micrometres and about 130 micrometres.

Where the sheet forming one or both of the peripheral portion and the support element is an aluminium sheet, the sheet may have a thickness of between about 10 micrometres and about 20 micrometres. An aluminium sheet with such thickness may advantageously make the hollow tubular element easier to manufacture, for example, by making the sheet easier to at least one of roll, bend and fold the sheet. In addition, an aluminium sheet with such thickness may provide the hollow tubular element with sufficient strength and stiffness to prevent or resist movement of one or more components disposed upstream of the hollow tubular element, such as the capsule, whilst preventing deformation of the hollow tubular element. Furthermore, an aluminium sheet with such basis weight may advantageously ensure that the hollow tubular element has a desired porosity in the longitudinal direction.

Substantially the entirety of the support element may be formed from a single layer of the sheet which forms the support element. In this case, substantially the entirety of the support element may have a thickness about the same as the thickness of the sheet. The support element may comprise a seam, the seam may be formed from overlapped layers of the sheet. The overlapped layers of the sheet forming the seam may be attached to each other by an adhesive.

The peripheral portion of the hollow tubular element may be formed from a sheet. The peripheral portion may be formed from a single layer of the sheet. The peripheral portion may be formed from a plurality of overlapping layers of the sheet, such as a plurality of parallel wound sheet layers or a plurality of spirally wound sheet layers. Where the peripheral portion comprises a seam, the seam may be formed from overlapped layers of the sheet. For example, the majority of the peripheral portion may be formed from a single layer of the sheet, and the seam may be formed from two overlapped layers of the sheet.

Where the peripheral portion is formed from a single layer of a sheet, the peripheral portion has a thickness about the same as the thickness of the sheet.

The peripheral portion may be formed from multiple sheets. For example, the peripheral portion may be formed from both a sheet which forms the support element, and an additional sheet.

The peripheral portion may be formed from a total of four layers or less of one or more of the sheets that form the peripheral portion. The peripheral portion may be formed from a combined total of four layers or less of the sheets that form the peripheral portion.

A section of the peripheral portion may be formed from a different number of layers of a sheet from a further section of the peripheral portion. For example, a section of the peripheral portion may be formed from one layer of a sheet, and an additional section of the peripheral portion may be formed from two layers of the sheet. As another example, a section of the peripheral portion may be formed from two layers of a sheet, an additional section of the peripheral portion may be formed from three layers of the sheet, and a further section of the peripheral portion may be formed from four layers of the sheet.

The peripheral portion may have a thickness of about 15 micrometres or more, about 45 micrometres or more, about 100 micrometres or more. Providing a peripheral portion with such thickness may provide the hollow tubular element with sufficient strength and stiffness to prevent or resist movement of one or both of the first element and the susceptor element, whilst preventing deformation of the hollow tubular element.

The peripheral portion may have a thickness of about 600 micrometres or less, about 500 micrometres or less, about 400 micrometres or less. Providing a peripheral portion with such thickness may advantageously ensure that the hollow tubular element has a desired porosity in the longitudinal direction. This may be such that the hollow tubular element has a desired resistance to draw. In addition, providing a peripheral portion with such thickness may mean that individual hollow tubular elements may be easily cut from a continuous rod of hollow tubular element. This may simplify manufacturing of the hollow tubular element.

The peripheral portion may have a thickness of between about 15 micrometres and about 600 micrometres, between about 50 micrometres and about 500 micrometres, between about 100 micrometres and about 400 micrometres. Preferably, the peripheral portion has a thickness of between about 100 micrometres and about 130 micrometres.

The inventors have found that a hollow tubular element having a hardness of at least about 90 percent may enable the hollow tubular element to prevent or restrict movement of one or more components disposed upstream of the hollow tubular element, such as the capsule, whilst avoiding significant deformation during interaction of the inhaler article with a holder.

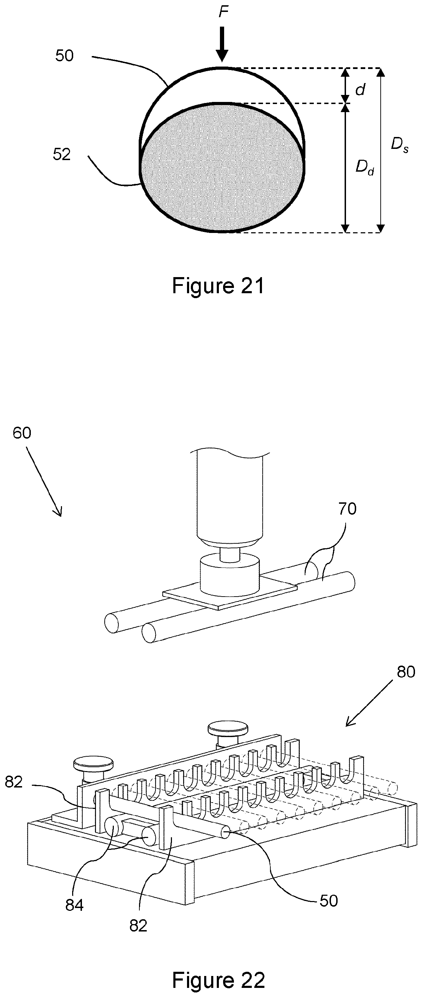

As used herein, the term "hardness" denotes the resistance to deformation. Hardness is generally expressed as a percentage. Figure 21 shows a hollow tubular element 50 before applying a load F and the same hollow tubular element 52 whilst applying load F. The hollow tubular element 50 before load F has been applied has an outer diameter $D_S$. The hollow tubular element 52 after applying a set load for a set duration (but with the load still applied) has a (reduced) outer diameter $D_d$. The depression is d = $D_S$ - $D_d$. Referring to Figure 21, hardness is given by:

$$hardness\ (\%) = \frac{D_d}{D_S}*100\%$$

Where $D_S$ is the original (undepressed) hollow tubular element outer diameter, and $D_d$ is the depressed outer diameter after applying a set load for a set duration. The harder the hollow tubular element, the closer the hardness is to 100%.

As is described in more detail below, to determine the hardness of a hollow tubular element, hollow tubular elements should be aligned parallel in a plane and the same portion of each hollow tubular element to be tested should be subjected to a set load for a set duration. The test is the DD60A Test and is performed using a known DD60A Densimeter device (manufactured and made commercially available by Heinr. Borgwaldt GmbH, Germany), which is fitted with a measuring head for hollow tubular elements and with a hollow tubular element receptacle.

The load is applied using two load applying cylindrical rods, which extend across the diameter of all of the hollow tubular elements at once. According to the standard test method for this instrument, the test should be performed such that twenty contact points occur between the hollow tubular elements and the load applying cylindrical rods. In some cases, the hollow tubular element to be tested may be long enough such that only ten hollow tubular elements are needed to form twenty contact points, with each hollow tubular element contacting both load applying rods (because they are long enough to extend between the rods). In other cases, if the hollow tubular elements are too short to achieve this, then twenty hollow tubular elements should be used to form the twenty contact points, with each hollow tubular element contacting only one of the load applying rods, as further discussed below.

Two further stationary cylindrical rods are located underneath the hollow tubular elements, to support the hollow tubular elements and counteract the load applied by each of the load applying cylindrical rods. Such an arrangement is described in more detail below, and shown in Figures 22 to 24.

For the standard operating procedure for such an apparatus, an overall load of 2 kg is applied for a duration of 20 seconds. After 20 seconds have elapsed (and with the load still being applied to the hollow tubular elements), the depression in the load applying cylindrical rods is determined, and then used to calculate the hardness from the above equation. The temperature is kept in the region of 22 degrees Centigrade ± 2 degrees. The test described above is referred to as the DD60A Test. The DD60A Test and corresponding apparatus are described in more detail below in relation to Figures 22 to 24. The hardness of a hollow tubular element of an inhaler article may not greatly differ between a hollow tubular element in an inhaler article that has been consumed and a hollow tubular element in an unused inhaler article.

However, the standard way to measure the hardness of a hollow tubular element is when the hollow tubular element is not part of an inhaler article that has been consumed.

The hardness of the hollow tubular element may be at least about 90%. Preferably, the hardness of the hollow tubular element is at least about 92%. This provides even better resistance to movement for one or more components disposed upstream of the hollow tubular element, such as the capsule. This also provides even better resistance to deformation for the hollow tubular element during interaction of the inhaler article with a holder.

A hollow tubular element with a low overall weight has the advantage that it can be assembled in an inhaler article using high speed machines and processes. In particular, the inventors of the present disclosure have found that a hollow tubular element with an overall weight of about 150 milligrams or less can advantageously be assembled in an inhaler article using existing high speed inhaler article assembly machines.

The hollow tubular element may have an overall weight of about 150 milligrams or less, preferably about 100 milligrams or less, more preferably about 70 milligrams or less.

The hollow tubular element may have an overall weight of between about 15 milligrams and about 150 milligrams, preferably between about 20 milligrams and about 100 milligrams, about 25 milligrams and about 70 milligrams.

The hollow tubular element may have an overall weight of about 34 milligrams. The hollow tubular element may have an overall weight of about 76 milligrams.

The hollow tubular element may have an average weight of about 10 milligrams per millimetre length of the hollow tubular element or less, preferably about 8 milligrams per millimetre length of the hollow tubular element or less, more preferably about 6 milligrams per millimetre length of the hollow tubular element or less. Providing a hollow tubular element with such average weight may advantageously enable the hollow tubular element to be assembled into an inhaler article using existing high speed inhaler article assembly machines.

The hollow tubular element may have an average weight of between about 1 and about 10 milligrams per millimetre length of the hollow tubular element, preferably between about 1.5 and about 8 milligrams per millimetre length of the hollow tubular element, more preferably between about 2 and about 6 milligrams per millimetre length of the hollow tubular element.

The hollow tubular element may have an average weight of about 4.25 milligrams per millimetre length of the hollow tubular element.

As used herein, the average weight of the hollow tubular element is measured by dividing the total weight of the hollow tubular element by the length of the hollow tubular element.

Part of the hollow tubular element may be circumscribed by a wrapper. The entirety of the hollow tubular element may be circumscribed by a wrapper. The wrapper may be a paper wrapper.

Preferably, the hollow tubular element is connected to one or more of the adjacent components of the inhaler article by means of a wrapper. The wrapper may be a paper wrapper.

The hollow tubular element may comprise an adhesive.

For example, where the peripheral portion comprises a tube, the sheet forming the support element may be attached to the tube by an adhesive at points where the sheet is in contact with the tube. As another example, a point at the peripheral portion may be attached to another point at the peripheral portion by an adhesive. For instance, the first point at the peripheral portion may be attached to the second point at the peripheral portion by an adhesive As another instance, where the sheet which forms the support element also forms part of the peripheral portion, the portion of the sheet which forms part of the peripheral portion may be attached to the remainder of the peripheral portion by an adhesive. As a further example, where the support element is in contact with the peripheral portion, the support element may be attached to the peripheral portion at a point of contact by an adhesive. For instance, where the support element comprises an end of the sheet, the end of the sheet may be attached to the peripheral portion by an adhesive. As an additional example, a point at the support element may be attached to another point at the support element. For instance, where the support element comprises a first side wall and a second side wall, the first side wall may be attached to the second side wall by an adhesive. In addition, where the hollow tubular element comprises a seam formed from overlapped layers of a sheet, the overlapped layer of the sheet may be attached to each other by an adhesive to form the seam.

The adhesive may comprise at least one of PVA, PVOH and hot melt glue.

The adhesive may comprise a binder. Suitable binders include, but are not limited to: gums such as, for example, guar gum, xanthan gum, arabic gum and locust bean gum; cellulosic binders such as, for example, hydroxypropyl cellulose, carboxymethyl cellulose, hydroxyethyl cellulose, methyl cellulose and ethyl cellulose; polysaccharides such as, for example, starches, organic acids, such as alginic acid, conjugate base salts of organic acids, such as sodium-alginate, agar and pectins; and combinations thereof. Preferably, the binder comprises guar gum.

The present disclosure also relates to a method for forming a hollow tubular element for an inhaler article. The method may comprise providing an apparatus for forming the hollow tubular element. The apparatus may comprise a device. The device may have an internal surface. The internal surface may define a channel of the device. The channel may extend from an upstream opening of the device. The channel may extend to a downstream opening of the device. The device may comprise an internal projection projecting into the channel. The method may also comprise providing a hollow tube. The method may further comprise passing the hollow tube into the channel through the upstream opening of the device. The method may further comprise passing the tube along the channel and into contact with the internal projection of the device, such that the tube is folded by the internal projection to form a hollow tubular element having a support element.

According to the present disclosure, there is a method comprising providing an apparatus for forming the hollow tubular element. The apparatus comprises a device. The device has an internal surface defining a channel. The channel extends from an upstream opening of the device to a downstream opening of the device. The device comprises an internal projection projecting into the channel. The method also comprises providing a hollow tube. The method further comprises passing the hollow tube into the channel through the upstream opening of the device; passing the tube along the channel and into contact with the internal projection of the device; such that the tube is folded by the internal projection to form a hollow tubular element having a support element.

The method may also comprise passing the hollow tubular element out of the channel through the downstream opening of the device.

The hollow tube may be formed from a sheet. The method may comprise forming the hollow tube from a sheet. Forming the hollow tube from a sheet may comprise forming a seam by overlapping a portion of the sheet at a first end of the sheet with a portion of the sheet at an opposed second end of the sheet. Forming the seam may comprise attaching the portion of the sheet at the first end of the sheet to the portion of the sheet at the second end of the sheet by an adhesive. The seam may extend along the length of the hollow tube.

A diameter of the hollow tube may be about the same as a perimeter of the hollow tubular element.

The channel may have a substantially circular cross section. The channel may comprise a substantially cylindrical section. The channel may comprise a substantially frustoconical section.

The internal projection may have a substantially constant cross section along the entire length of the internal projection. The internal projection may have a cross section that varies along the length of the internal projection. For example, the internal projection may taper. For instance, the internal projection may taper off at an upstream end of the internal projection. The length of the internal projection may extend in the direction that the hollow tube passes through the device.

The internal projection may have a substantially rectangular cross section in one or both of the longitudinal direction and the transverse direction. The internal projection may have a substantially triangular cross section in one or both of the longitudinal direction and the transverse direction. Preferably, the internal projection has a triangular cross section in the transverse direction. A triangular cross section in the transverse direction may assist with folding of the hollow tube to form a hollow tubular element, and may avoid tearing through the hollow tube. The internal projection may be substantially pyramidal.

Where the internal projection is substantially pyramidal, the internal projection may have a maximum transverse cross-sectional area at an apex of the internal projection.

Where the internal projection has a substantially triangular cross section in the transverse direction, for example when the internal projection is substantially pyramidal, the internal projection may comprise a first edge. The first edge may be adjacent to a portion of the internal surface of the device that defines the channel. The internal projection may comprise a second edge. The second edge may be adjacent to a portion of the internal surface of the device that defines the channel. The second edge may extend from an upstream end of the internal projection. The internal projection may comprise a third edge. The third edge may reside within the channel. The third edge may extend from the upstream end of the internal projection. The third edge may extend to an apex of the internal projection. The third edge may define a tip of the internal projection.

The hollow tube may have a circumference about equal to the internal perimeter of a transverse cross section of the device at the apex of the internal projection.

The internal projection may be a first internal projection and the device may comprise one or more additional internal projections. The device may comprise between two and six internal projections. Preferably, the device comprises three internal projections. Each of the internal projections may be identical to one another. Alternatively, one of the internal projections may be different to another internal projection. The internal projections may be equally spaced around the channel.

The internal shape of the device may be configured such that a snug fit is achieved between the hollow tube and the internal surface of the device defining the channel. This may be particularly desirable at points where the hollow tube is in contact with one or more of the internal projections. This may help with folding of the hollow tube at desired positions to form a hollow tubular element.

The device may comprise a first section. The first section of the device may comprise at least part of the channel of the device. The channel may have a substantially constant cross section along the entire length of the first section of the device. For instance, the part of the channel extending through the first section of the device may be substantially cylindrical. A cross section of the channel may vary along the length of the first section of the device. For instance, a cross-sectional area of the channel at an upstream end of the first section of the device may be larger than a cross-sectional area of the channel at a downstream end of the first section of the device. Preferably, the part of the channel extending through the first section of the device is substantially frustoconical. Where this is the case, preferably the diameter of the channel of the device at the upstream end of the first section is greater than the diameter of the channel of the device at the downstream end of the first section. A diameter of the channel of the device at a point along the first section, for example at the upstream end of the first section, may be about the same as a diameter of the hollow tube. The diameter of the channel at a point along the first section, for example at the downstream end of the first section, may be about the same as the diameter of the hollow tubular element. The diameter of the channel may be selected such that an outer surface of the hollow tube remains in contact with an inner surface of the device, during a step of passing the hollow tube through the first section of the device, to assist with shaping of the hollow tube into a hollow tubular element.

The internal projection may be a part of the first section of the device. That is, the first section of the device may comprise the internal projection projecting into the channel. The internal projection may extend from an upstream end of the first section of the device to a downstream end of the first section of the device. As such, the internal projection may extend along the entire length of the first section of the device. The internal projection may project into the part of the channel that extends through the first section of the device. Where the internal projection tapers, the internal projection may taper off at the upstream end of the first section of the device. In addition, where the internal projection comprises a first edge, the first edge may extend from the upstream end of the first section of the device. Where the internal projection comprises a section edge, the second edge may extend from the upstream end of the first section of the device. Where the internal projection comprises a third edge, the third edge may extend from the upstream end of the first section of the device. The third edge may reside within the channel.

The first section of the device may extend from the upstream opening of the device to the downstream opening of the device. In this case, the first section of the device may be the only section of the device. That is, the device may comprise only the first section of the device.

In addition to the first section, the device may comprise one or more additional sections.

For example, the device may comprise a second section. The second section of the device may comprise at least part of the channel of the device. The second section may extend from the upstream opening of the device. The second section may extend to the first section of the device. In other words, the second section may be adjacent to, and upstream from, the first section of the device.

The part of the channel extending through the second section may have a substantially circular cross section. Preferably, the part of the channel extending through the second section has a substantially circular cross section at the downstream end of the second section. Where this is the case, preferably a diameter of the channel at a downstream end of the second section is about the same as a diameter of the channel at the upstream end of the first section.

The channel may have a larger cross sectional area at the upstream end of the second section than at the downstream end of the second section. The part of the channel extending through the second section may be substantially frustoconical.

The part of the channel extending through the second section may have a substantially constant cross section along the entire length of the second section. The part of the channel extending through the second section may be substantially cylindrical.

The device may comprise a third section. The third section of the device may comprise at least part of the channel of the device. The third section may extend from the downstream end of the first section of the device. The third section may extend to the downstream opening of the device. In other words, the third section may be adjacent to, and downstream from, the first section of the device.

The part of the channel extending through the third section may have a substantially circular cross section. Preferably, the part of the channel extending through the third section has a substantially circular cross section at the upstream end of the third section. Where this is the case, preferably a diameter of the channel at an upstream end of the third section is about the same as a diameter of the channel at the downstream end of the first section.

The channel may have a larger cross sectional area at the downstream end of the third section than at the upstream end of the third section. The part of the channel extending through the third section may be substantially frustoconical.

The part of the channel extending through the third section may have a substantially constant cross section along the entire length of the third section. The part of the channel extending through the third section may be substantially cylindrical.

The device may comprise only the first section and the third section. The device may comprise a first section, a second section and a third section. Where this is the case, the first section may be located between the second section and the third section of the device.

The method comprises passing the hollow tube into the channel of the device through the upstream opening of the device.

The method also comprises passing the hollow tube along the channel and into contact with the internal projection of the device. Where the device comprises a first section comprising the internal projection, the method may comprise passing the hollow tube along the channel and into contact with the internal projection at the upstream end of the first section of the device. The method may also comprise passing the hollow tube along the channel through the first section of the device, such that an outer surface of the hollow tube is in contact with the internal surface of the first section of device. The method may also comprise passing the hollow tube along the channel through the first section of the device, such that an outer surface of the hollow tube is in contact with the internal projection. Due to the configuration of the first section of the device, passing the hollow tube along the first section of the device may cause the hollow tube to deform and conform to the internal shape of the first section of the device. In particular, where the part of the channel extending through the first section has a substantially frustoconical shape, the shape of the channel in the first section combined with the presence of the internal projection in the first section, may help to shape the hollow tube into a form having a reduced diameter and an internal folded projection forming a support element. Consequently, passing the hollow tube through the first section of the device may cause the hollow tube to form: a first fold line at a first edge of the internal projection, a second fold line at a second edge of the internal projection; and a third fold line at a third edge of the internal projection. As such, passing the hollow tube through the first section of the device may form a hollow tubular element formed from a sheet, the hollow tubular element comprising: a peripheral portion defining a hollow inner region, and a support element; wherein the support element depends from the peripheral portion along both a first fold line of the sheet and a second fold line of the sheet; and wherein the support element comprises a third fold line of the sheet residing within the hollow inner region.

The method may comprise passing the hollow tubular element out of the channel through the downstream opening of the device.

Where the device comprises a second section extending from the upstream opening of the device to the upstream end of a first section of the device, the method comprises passing the hollow tube through the second section of the device, along the channel, prior to passing the hollow tube through the first section of the device. Passing the hollow tube through the second section of the device may assist with insertion of the hollow tube into the channel and into contact with the internal projection.

Where the device comprises a third section extending from the downstream end of a first section of the device to the downstream opening of the device, the method may comprise passing the hollow tube through the third section of the device, along the channel, following passing the hollow tube through the first section of the device. The method may comprise passing the hollow tubular element through the third section of the device and out of the channel through the downstream opening of the device. Passing the hollow tubular element through the third section of the device may also assist with the exiting of the hollow tubular element out of the device. Passing the hollow tubular element through the third section of the device may help to retain the desired shape of the hollow tubular element after folding of the hollow tubular element, for example, by helping to retain the desired curvature of the hollow tubular element.

The method may comprise attaching a first side wall of the support element to a second side wall of the support element by an adhesive, where the first side wall of the support element extends from the first fold line to the third fold line, and the second side wall of the support element extends from the second fold line to the third fold line. The attaching step may be performed before the hollow tubular element has exited the device. In this case, the attaching step may be performed whilst the hollow tubular element is being passed through the channel. The attaching step may be performed after the hollow tubular element has exited the device.

The method may comprise circumscribing a wrapper around the hollow tubular element. The circumscribing step may be performed before the hollow tubular element has exited the device. The circumscribing step may be performed after the hollow tubular element has exited the device.

The method may comprise attaching a wrapper to the hollow tubular element, for example, by an adhesive. The step of attaching a wrapper to the hollow tubular element may be performed before the hollow tubular element has exited the device. The step of attaching a wrapper to the hollow tubular element may be performed after the hollow tubular element has exited the device.

Features described in relation to one example or embodiment may also be applicable to other examples and embodiments.

Embodiments of the disclosure will now be described in detail, by way of example only, with reference to the accompanying drawings, in which:

Figure 1 shows a schematic side sectional view of an inhaler article in accordance with a first embodiment of the present disclosure;

Figure 2 shows a cutaway perspective view of the inhaler article of Figure 1;

Figure 3 shows a partially transparent perspective view of a hollow tubular element of the inhaler article of Figure 1;

Figures 4A and 4B show a cross-sectional view of the upstream end face of the hollow tubular element of the inhaler article of Figure 1;

Figure 4C shows a cross-sectional view of the inhaler article at the hollow tubular element of Figure 1;

Figure 5 shows a perspective view of a hollow tubular element for an inhaler article in accordance with a second embodiment of the present disclosure;

Figure 6 shows a cross-sectional view of the upstream end face of the hollow tubular element of Figure 5;

Figure 7 shows a cross-sectional view of the upstream end face of a hollow tubular element for an inhaler article in accordance with a third embodiment of the present disclosure;

Figure 8 shows a cross-sectional view of the upstream end face of a hollow tubular element for an inhaler article in accordance with a fourth embodiment of the present disclosure;

Figure 9 shows a cross-sectional view of the upstream end face of a hollow tubular element for an inhaler article in accordance with a fifth embodiment of the present disclosure;

Figure 10 shows a side view of an apparatus for forming a hollow tubular element for an inhaler article, for example, in accordance with the first embodiment of the present disclosure;

Figure 11A shows a cross-sectional view of the apparatus of Figure 10 as taken along plane A-A of Figure 10;

Figure 11B shows a cross-sectional view of the apparatus of Figure 10 as taken along plane B-B of Figure 10;

Figure 12A shows a cross-sectional view of a hollow tube used to form a hollow tubular element for an inhaler article, for example, in accordance with the first embodiment of the present disclosure;

Figure 12B shows a cross-sectional view of a hollow tubular element for an inhaler article formed from the hollow tube of Figure 12A and using the apparatus of Figure 10;

Figure 13 shows a perspective view of a hollow tubular element for an inhaler article in accordance with a sixth embodiment of the present disclosure;

Figure 14 shows a cross-sectional view of the upstream end face of the hollow tubular element of Figure 13;

Figure 15 shows a cross-sectional view of the upstream end face of a hollow tubular element for an inhaler article in accordance with a seventh embodiment of the present disclosure;

Figure 16 shows a cross-sectional view of the upstream end face of a hollow tubular element for an inhaler article in accordance with an eight embodiment of the present disclosure;

Figure 17 shows a cross-sectional view of the upstream end face of a hollow tubular element for an inhaler article in accordance with a ninth embodiment of the present disclosure;

Figure 18 shows a perspective view of a hollow tubular element for an inhaler article in accordance with a tenth embodiment of the present disclosure;

Figure 19 shows a cross-sectional view of the upstream end face of the hollow tubular element of Figure 18;

Figure 20 shows a cross-sectional view of the upstream end face of a hollow tubular element for an inhaler article in accordance with an eleventh embodiment of the present disclosure;

Figure 21 shows a cross-sectional view of a hollow tubular element both before and after a load has been applied for determining the hardness of the hollow tubular element;

Figure 22 shows a perspective view of an apparatus for determining the hardness of a hollow tubular element for a smoking article, in a first configuration;

Figure 23 illustrates a side view of the apparatus of Figure 22, in a first configuration;

Figure 24 illustrates a side view of the apparatus of Figure 22, in a second configuration;

Figure 25 shows a cutaway perspective view of an inhaler article comprising the hollow tubular element of Figure 18;

Figure 26 shows a schematic side sectional view of an inhaler system;

Figure 27 shows a schematic side sectional view of the sleeve of the inhaler system of Figure 26; and

Figure 28 shows a schematic side sectional view of the inhaler article of Figure 18 received in the sleeve of Figure 27.

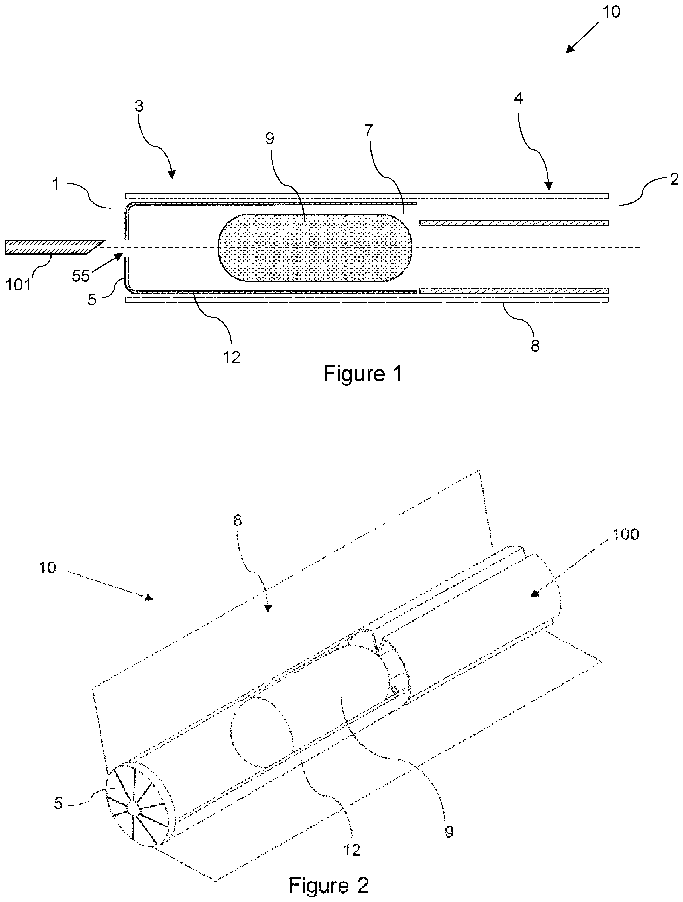

Figures 1 and 2 illustrate an inhaler article 10 in accordance with the present disclosure. The inhaler article 10 extends between its upstream end 1 and its downstream (or mouth) end 2. The inhaler article 10 comprises an upstream section 3 and a downstream section 4 located downstream of the upstream section 3 and spaced apart from the upstream section 3. A cavity 7 configured to receive a capsule 9 containing inhalable material is between the upstream and downstream sections 3, 4 of the inhaler article 10. The inhalable material comprises nicotine.

As shown in Figures 1 and 2, the upstream section 3 comprises a folded end 5 of a hollow tube 12 and the downstream section 4 comprises a hollow tubular element 100. The hollow tubular element 100 extends from the cavity 7, or the downstream portion thereof, to the downstream end 2 of the inhaler article 10.

The inhaler article 10 further comprises an overall wrapper 8 wrapping both the hollow tube 12 and the hollow tubular element 100. The hollow tube 12 contains the cavity 7. The hollow tube 12 and the upstream end of the hollow tubular element 100 define the cavity 7. The downstream end of the hollow tube 12 abuts the upstream end of the hollow tubular element 100 of the downstream section 4. The hollow tubular element 100 forms the downstream section 4. The overall wrapper 8 circumscribes both the hollow tube 12 and the downstream section 4. The wrapper 8 secures the downstream section 4 in axial alignment with the hollow tube 12.

In the embodiment shown in Figures 1 and 2, the overall length of the inhaler article 10 is about 45 mm. The length of the cavity 7 is about 28 mm and the length of the hollow tubular element 100 is about 17 mm. The length of the hollow tube 12 surrounding the cavity 7 is between about 25 mm and about 28 mm. The inner diameter of the hollow tube 12 is about 6.6 mm and the outer diameter of the hollow tube 12 is about 7.1 mm. The length of the wrapping material 8 is about 45 mm. The diameter of the inhaler article 10 is about 7 mm. The relative RTD, or RTD per unit length, of the hollow tubular element 100 is about 0.02 mm of water per mm. The RTD of the hollow tubular element 100 is about 0.34 mm of water. A diameter of the capsule 9 is about 6 mm and the length of the capsule 9 is about 16 mm.

The folded end 5 of the hollow tube 12 defines a central channel or passage 55 extending through the centre of the folded end 5 from the upstream end of the folded end 5.

The central channel 55 of the folded end 5 is arranged to provide access to the cavity 7 to a piercing element 101, for example, a piercing element 101 of a holder for the inhaler article 10. Such a piercing element is configured to pierce or puncture the capsule 9 in order to activate it for consumption. A diameter of the central channel 55 is less than about 6mm. The central channel is structured to accommodate a piercing element or needle from 27 gauge (outer diameter = 0.42 mm) to 4 gauge (outer diameter = 5 mm).

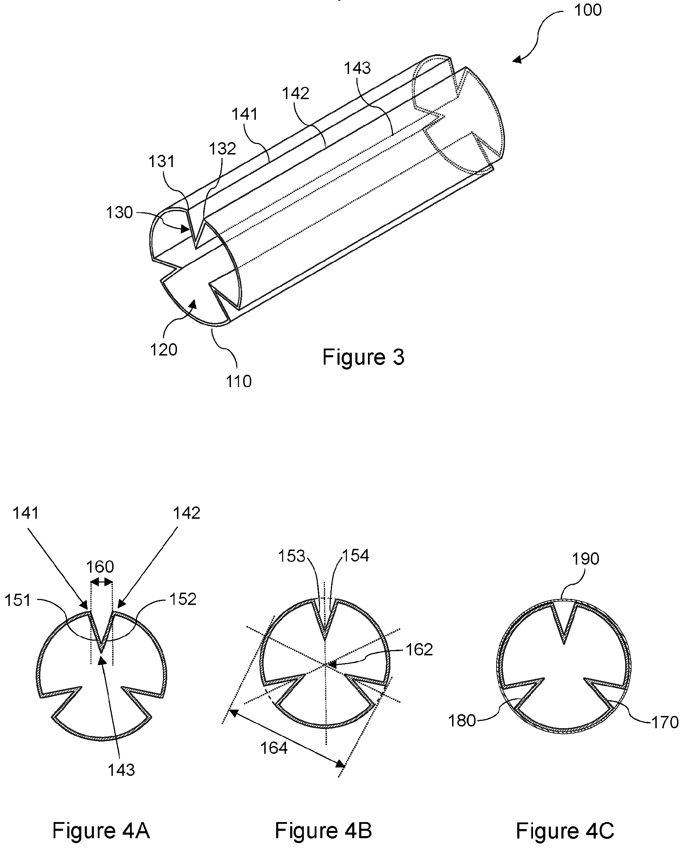

As best seen from Figure 3, the hollow tubular element 100 of the first embodiment comprises a peripheral portion 110 of material defining a hollow inner region 120 of the hollow tubular element 100. The hollow tubular element 100 also comprises a support element 130 formed from a sheet and extending from a first point 131 at the peripheral portion 110 across the hollow inner region 120 to a second point 132 at the peripheral portion 110.

The peripheral portion 110 and the support element 130 are integrally formed from the same sheet of paper. The paper sheet has a basis weight of about 78 grams per square metre. Substantially the entirety of the portion of the sheet forming the peripheral portion 110 forms a curved outer surface of the hollow tubular element 100.

To form the support element 130 the paper sheet comprises a seam (not shown), wherein two layers of the paper sheet overlap each other. The seam may be a part of one or both of the peripheral portion 110 and the support element 130. The seam extends over a small portion of one or both of the peripheral portion 110 and the support element 130. As such, substantially the entirety of the peripheral portion 110 is formed from a single layer of the sheet. In addition, substantially the entirety of the support element 130 is formed from a single layer of the sheet.

The support element 130 depends from the peripheral portion 110 along a first fold line 141 of the sheet, wherein the first fold line 141 resides at the first point 131 at the peripheral portion 110, and wherein the first fold line 141 extends along substantially the entire length of the hollow tubular element 100. The support element 130 also depends from the peripheral portion 110 along a second fold line 142 of the sheet, wherein the second fold line 142 resides at the second point 132 at the peripheral portion 110, and wherein the second fold line 142 extends along substantially the entire length of the hollow tubular element 100.

As such, the support element 130 also extends along substantially the entire length of the hollow tubular element 100. In effect, the support element 130 has substantially the same length as the hollow tubular element 100.

The hollow tubular element 100 has a length of about 17 millimetres.

The hollow tubular element 100 has a total weight of about 72 milligrams. As such, the hollow tubular element has an average weight of about 4.2 milligrams grams per millimetre.

The hollow tubular element 100 has a constant cross section along the entire length of the hollow tubular element 100.

The first fold line 141 and the second fold line 142 are both parallel to the longitudinal axis of the hollow tubular element 100. As such, the first fold line 141 and the second fold line 142 are parallel to each other.

As illustrated in Figure 3, the support element 130 comprises a third fold line 143 of the sheet, wherein the third fold line 143 is parallel to and equidistant between the first fold line 141 and the second fold line 142. This helps to provide a strong support barrier to prevent or reduce movement of the capsule 9, for example, when the capsule is being pierced by a piercing element. The third fold line 143 defines the tip of the support element.

Figures 4A and 4B show a cross-sectional view of the upstream end face of the hollow tubular element 100.

The first fold line 141 and the third fold line 143 together define a first side wall 151 of the support element 130, wherein the first side wall 151 is substantially straight and the outer surface 153 of the first side wall 151 forms an outer surface of the hollow tubular element 100. The second fold line 142 and the third fold line 143 together define a second side wall 151 of the support element 130, wherein the second side wall 152 is substantially straight and the outer surface 154 of the second side wall 152 forms an outer surface of the hollow tubular element.

The support element 130 has a generally triangular cross section.

The first point 131 at the peripheral portion 110 and the second point 132 at the peripheral portion 110 are spaced apart from each other by a distance 160 of about 1 millimetre. As such, the first fold line 141 and the second fold line 142 are also spaced apart from each other by a distance of about 1 millimetre.

The first side wall 151 and the second side wall 152 define an angle of about 30 degrees therebetween.

The depth of the support element 130 is about 2 millimetres. That is, the distance between the first point 131 at the peripheral portion and the tip of the support element 130 is about 2 millimetres. As such, the distance between the first fold line 141 and the third fold line 143 is also about 2 millimetres.

The tip of the support element 130 is spaced apart from the radial centre 162 of the hollow tubular element 100 by a distance of about 1.5 millimetres. As such, the support element 130 is spaced apart from the radial centre 162 of the hollow tubular element 100 by a distance of about 1.5 millimetres.

The outer diameter 164 of the hollow tubular element is about 7.2 millimetres. As such, the support element 130 is spaced apart from the radial centre 162 of the hollow tubular element 100 by a distance of about 42 percent of the radius of the hollow tubular element 100.

Figure 4C shows a wrapper 190 circumscribing the hollow tubular element 100.

The support element 130 is a first support element 130 and the hollow tubular element comprises two additional support elements: a second support element 170 and a third support element 180. This advantageously provides the hollow tubular element 100 with additional strength and stiffness in both the longitudinal direction and the transverse direction to prevent or restrict movement of the capsule 9, for example, when the capsule 9 is being pierced by a piercing element.

Each of the support elements 130, 170, 180 are identical to one another and are equally spaced around the circumference of the hollow tubular element 100. The circumference of the hollow tubular element 100 is illustrated by the dashed curved lines in Figure 4B.

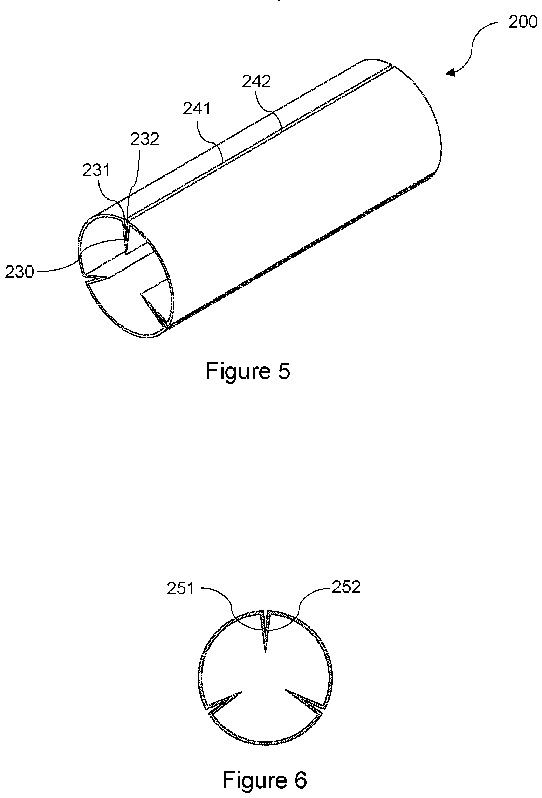

Figure 5 shows a perspective view of a hollow tubular element 200 for an inhaler article in accordance with a second embodiment of the present disclosure. The hollow tubular element 200 of the second embodiment differs from the hollow tubular element 100 of the first embodiment in that the first point 231 at the peripheral portion and the second point 232 at the peripheral portion are positioned closer to one another. In particular, the first point 231 at the peripheral portion and the second point 232 at the peripheral portion are spaced apart from each other by a distance of about zero millimetres. As such, the first fold line 241 and the second fold line 242 are also spaced apart from each other by a distance of about zero millimetres. The depth of the support element 230 is the same as the depth of the support element 130 and is about 2 millimetres.

Figure 6 shows a cross-sectional view of the upstream end face of the hollow tubular element 200. The angle formed between the first side wall 251 and the second side wall 252 is approximately zero degrees. Substantially the entirety of the first side wall 251 and substantially the entirety of the second side wall 252 are in contact with each other and are attached to each other by an adhesive. This can significantly increases the strength and the stiffness of the hollow tubular element in both the longitudinal direction and the transverse direction. This can also avoids the need to circumscribe the hollow tubular element 200 with a wrapper. As such, this can minimise the weight of the hollow tubular element 200 such that it is able to be assembled in the inhaler article 10 using existing high speed inhaler article assembly machines.

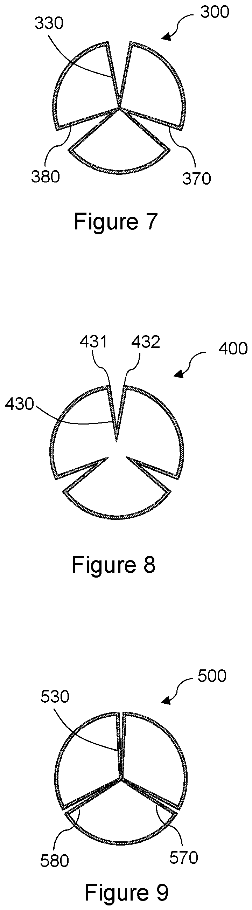

Figure 7 shows a cross-sectional view of the upstream end face of a hollow tubular element 300 for an inhaler article in accordance with a third embodiment of the present disclosure. The hollow tubular element 300 of the third embodiment is generally the same as the hollow tubular element 100 of the first embodiment. However, the hollow tubular element 300 of the third embodiment differs from the hollow tubular element 100 of the first embodiment in that the support element 330 has a depth equal to about the radius of the hollow tubular element 300. As such, the support element 330 extends to the radial centre of the hollow tubular element 300. In particular, the tip of the support element 330 resides at or is adjacent to the radial centre of the hollow tubular element 300. In a similar manner to the hollow tubular element 100 of the first embodiment, the hollow tubular element 300 of the third embodiment comprises three identical support elements 330, 370, 380 equally spaced around the circumference of the hollow tubular element 300. As such, the support elements 330, 370, 380 divide the hollow inner region into three channels. In particular, the tips of the support elements 330, 370, 380 are adjacent to one another at the radial centre of the hollow tubular element 300.

Figure 8 shows a cross-sectional view of the upstream end face of a hollow tubular element 400 for an inhaler article in accordance with a fourth embodiment of the present invention. The hollow tubular element 400 is generally the same as the hollow tubular element 400 of the first embodiment, with the exception that the first point 431 at the peripheral portion and the second point 432 at the peripheral portion are positioned closer to one another. In particular, the first point 431 at the peripheral portion and the second point 432 at the peripheral portion are spaced apart from each other by a distance of about 0.8 millimetres. Furthermore, in Figure 8, the depth of the support element 430 is now about 3 millimetres. In addition, in Figure 8, the first side wall and the second side wall define an angle of about 15 degrees therebetween.

Figure 9 shows a cross-sectional view of the upstream end face of a hollow tubular element 500 for an inhaler article in accordance with a fifth embodiment of the present disclosure. The hollow tubular element 500 is generally the same as the hollow tubular element 200 of the second embodiment, with the exception that the depth of the hollow tubular element 200 is about the same as the radius of the hollow tubular element 500. As such, the support element 530 extends to the radial centre of the hollow tubular element 500. In particular, the tip of the support element 530 resides at or is adjacent to the radial centre of the hollow tubular element 500. Similarly to the hollow tubular element 100 of the first embodiment and the hollow tubular element 200 of the second embodiment, the hollow tubular element 500 of the fifth embodiment comprises three identical support elements. As such, the three support elements of the hollow tubular element 500 divides the hollow region of the hollow tubular element 500 into three channels. In particular, the tips of the support elements 530, 370, 580 are adjacent to one another at the radial centre of the hollow tubular element 300.

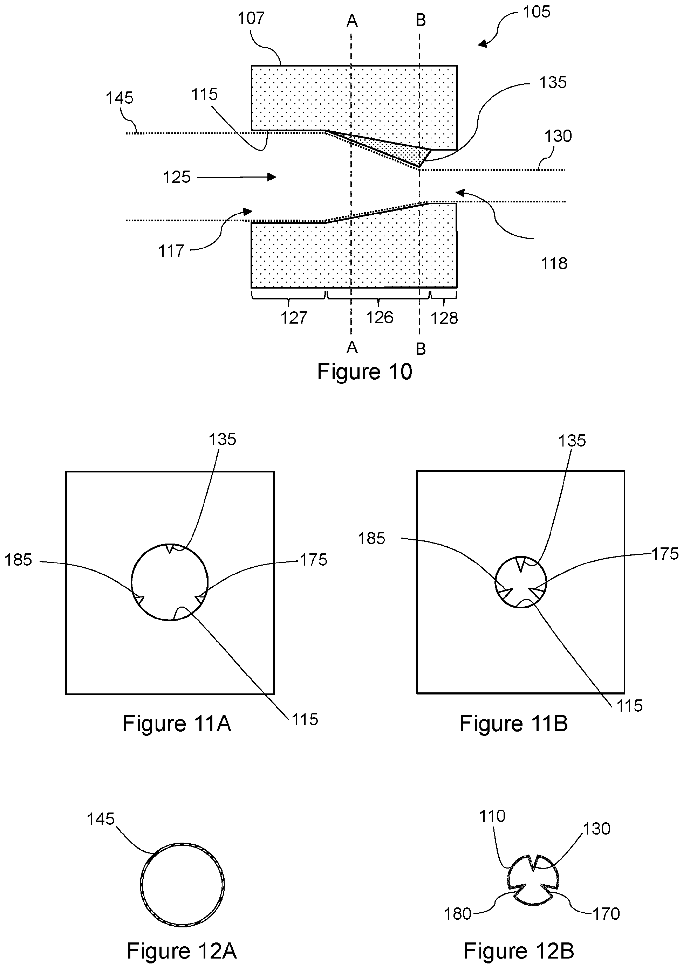

Figure 10 illustrates a method for forming a hollow tubular element for an inhaler article, such as the hollow tubular element 100 of the first embodiment described above. The method comprises providing an apparatus 105 for forming the hollow tubular element. The apparatus 105 comprises a device 107. The device 107 has an internal surface 115 defining a channel 125. The channel 125 extends from an upstream opening 117 of the device 107 to a downstream opening 118 of the device 107.

The device 107 comprises a first section 126, a second section 127 and a third section 128. The first section is located between the second section 127 and the third section 128, as shown in Figure 10.

The first section 126 of the device 107 comprises an internal projection 135 projecting into the channel 125. The internal projection 135 extends from an upstream end of the first section 126 of the device 107 to a downstream end of the first section 126 of the device 107. The channel 125 in the first section 126 of the device 107 is substantially frustoconical, wherein a diameter of the channel 125 at the upstream end of the first section 126 is greater than the diameter of the channel 125 at the downstream end of the first section 126.

The internal projection 135 is substantially pyramidal. The internal projection 125 has a substantially triangular cross section in both the longitudinal direction and the transverse direction. The internal projection 135 has a maximum transverse cross-sectional area at an apex of the internal projection 135 and tapers off at the upstream end of the first section 126 of the device 107. The internal projection comprises a first edge, wherein the first edge is adjacent to a portion of the internal surface of the device 107 that defines the channel 125. The first edge extends from the upstream end of the first section 126 of the device 107. The internal projection also comprises a second edge, wherein the second edge is also adjacent to the internal surface 115 of the device 107 that defines the channel. The second edge extends from the upstream end of the first section 126 of the device 107. The internal projection further comprises a third edge, wherein the third edge resides within the channel 125 and also extends from the upstream end of the first section 126 of the device 107.

A cross section of the internal projection 135 taken along plane A-A is shown in Figure 11A. A cross section of the internal projection 135 taken along plane B-B is shown in Figure 11B. As such, Figure 11B shows a cross section of the internal projection 135 at the apex of the internal projection 135.

The second section 127 of the device 107 extends from the upstream opening 117 of the device 107 to the first section 126 of the device 107. The part of the channel 125 extending through the second section 127 of the device 107 is substantially cylindrical and has a diameter about the same as the diameter of the channel 125 at the upstream end of the first section 126.

The third section 128 of the device 107 extends from the first section 126 of the device 107 to the downstream opening 118 of the device 107. The part of the channel 125 extending through the third section 128 of the device 107 is substantially cylindrical and has a diameter about the same as the diameter of the channel 125 at the downstream end of the first section 126.

The method also comprises providing a hollow tube 145 formed from a sheet, wherein a circumference of the hollow tube 145 is about equal to the internal perimeter of a transverse cross section of the device 107 at the apex of the internal projection 135. A cross section of the hollow tube 145 is shown in Figure 11 A. The diameter of the channel 125 at the upstream end of the first section 126 is about the same as a diameter of the hollow tube 145. As such, the diameter of the hollow tube 145 is also about the same as the diameter of the part of the channel 125 extending through the second section 127 of the device 107.

The method further comprises passing the hollow tube 145 through the upstream opening 117 of the device 107, into the second section 127 of the device 107, along the channel 125.

The method further comprises passing the hollow tube 145 along the channel 125 and into contact with the internal projection 135 at the upstream end of the first section 126 of the device 107.

The method further comprises passing the hollow tube 145 along the channel 125 through the first section 126 of the device 107, such that an outer surface of the hollow tube 145 is in contact with the internal surface 115 of the device 107. In particular, such that an outer surface of the hollow tube 145 is in contact with the internal projection 135. Due to the configuration of the first section 126 of the device 107, passing the hollow tube 145 along the first section 126 of the device 107 causes the hollow tube 145 to deform and conform to the internal shape of the first section of the device 107. In particular, the frustoconical shape of the channel 125 in the first section 126 when combined with the presence of the internal projection 135 in the first section 126, helps to shape the hollow tube 145 into a form having a reduced diameter and an internal folded projection forming a support element 130 as shown in Figure 12B. Consequently, passing the hollow tube 145 through the first section 126 of the device 107 causes the hollow tube 145 to form: a first fold line at the first edge of the internal projection 135, a second fold line at the second edge of the internal projection 135; and a third fold line at the third edge of the internal projection 135. As such, passing the hollow tube 145 through the first section 126 of the device 107 forms a hollow tubular element formed from a sheet, the hollow tubular element comprising: a peripheral portion 110 defining a hollow inner region, and a support element 130; wherein the support element 130 depends from the peripheral portion along both a first fold line of the sheet and a second fold line of the sheet; and wherein the support element comprises a third fold line of the sheet residing within the hollow inner region. The hollow tube 145 and the hollow tubular element are shown in dotted lines in Fig. 10.

The method further comprises passing the hollow tubular element through the third section 128 of the device 107 and out of the channel 117 through the downstream opening 118 of the device 107. The third section 128 of the device 107 may assist with the exiting of the hollow tubular element out of the device 107. In addition, the third section 128 of the device 107 may help to retain the desired shape of the hollow tubular element after folding of the hollow tubular element.

As shown in Figures 11A and 11B, the internal projection 135 is a first internal projection 135 and the first section 126 of the device 107 comprises two additional internal projections: a second internal projection 175 and a third internal projection 185. Each of the internal projections 135, 175, 185 are identical to one another and are equally spaced around the circumference of the first section 126 of the device 107.

As such, as shown in Figure 12B, the support element 130 of the hollow tubular element formed by passing the hollow tube 145 through the first section 126 of the device 107 is a first support element 130 and the hollow tubular element comprises two additional support elements: a second support element 170 and a third support element 180. Each of the support elements 130, 170, 180 are identical to one another and are equally spaced around the circumference of the hollow tubular element.

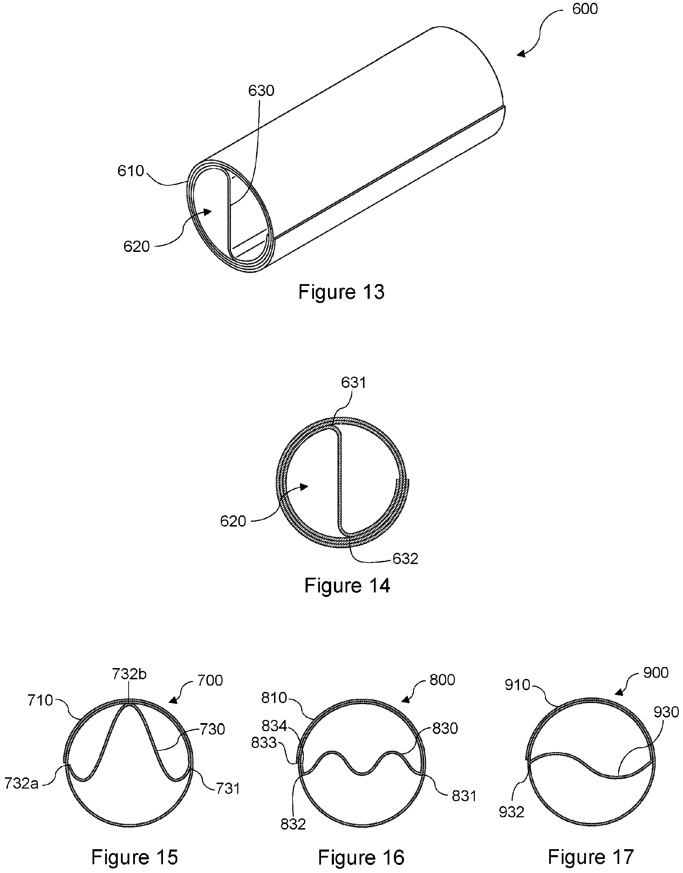

Figure 13 shows a perspective view of a hollow tubular element 600 for an inhaler article in accordance with a sixth embodiment of the present disclosure. The hollow tubular element 600 comprises a peripheral portion 610, which defines a hollow inner region 620 of the hollow tubular element 600; and a support element 630.

As shown in Figures 13 and 14, the peripheral portion 610 and the support element 630 are formed integrally from the same sheet of paper. In particular, the peripheral portion 610 is formed from between two and four parallel wound layers of the paper sheet, and the support element 630 is formed from a single layer of the paper sheet. More specifically, a section of the peripheral portion 610 is formed from two layers of the paper sheet, another section of the peripheral portion 610 is formed from three layers of the paper sheet, and a further section of the peripheral portion 610 is formed from four layers of the paper sheet.

As illustrated by Figure 14, the support element 630 extends from a first point 631 at the peripheral portion 610 across the hollow inner region 620 through the radial centre of the hollow tubular element 600 to a second point 632 at the peripheral portion 610. The first point 631 at the peripheral portion 610 and the second point 632 at the peripheral portion 610 are about diametrically opposed to each other. The inner diameter of the hollow tubular element is about 6.9 millimetres. As such, the first point 631 at the peripheral portion 610 and the second point 632 at the peripheral portion 610 is spaced apart from each other by about 6.9 millimetres. The outer diameter of the hollow tubular element is about 7.2 millimetres.

The support element 630 comprises a substantially straight portion which extends from the first point 631 at the peripheral portion 610 to the second point 632 at the peripheral portion 610, when viewed from the upstream end of the hollow tubular element 600, as shown in Figure 14.

The support element 630 depends from the peripheral portion 610 along a first fold line of the sheet, wherein the first fold line resides at the first point 631 at the peripheral portion 610. The support element 630 also depends from the peripheral portion 610 along a second fold line of the sheet, wherein the second fold line resides at the second point 632 at the peripheral portion 610. As such, the substantially straight portion also extends from the first fold line of the sheet to the second fold line of the sheet.

Figure 15 shows a cross-sectional view of the upstream end face of a hollow tubular element 700 for an inhaler article in accordance with a seventh embodiment of the present disclosure. The hollow tubular element 700 comprises a peripheral portion 710 and a support element 730. The peripheral portion 710 and the support element 730 are formed integrally from the same sheet of paper. The peripheral portion 710 is formed from parallel wound layers of the sheet such that a section of the peripheral portion is formed from two layers of the sheet and another section of the peripheral portion 710 is formed from a single layer of the sheet.

The support element 730 extends from a first point 731 at the peripheral portion 710 across the hollow inner region to a second point 732a at the peripheral portion 710. In particular, the support element 730 comprises an end of the sheet, wherein the end of the sheet is in contact with the peripheral portion 710 at the second point 732a at the peripheral portion 710.

The support element 730 is substantially sinusoidal, when viewed from the upstream end of the hollow tubular element 700. The support element 730 comprises a plurality of peaks and troughs; in particular, the support element 730 comprises a peak and two troughs. The peak of the support element 730 is in contact with the peripheral portion 710 at a further point 732b at the peripheral portion 710.

As such, it will be appreciated that the portion of the sheet extending from the first point 731 at the peripheral portion 710 to the further point 732b at the peripheral portion 710 may be a first support element. In addition, the portion of the sheet extending from the further point 732b at the peripheral portion 710 to the second point 732a at the peripheral portion 710 may be a second support element.

Figure 16 shows a cross-sectional view of the upstream end face of a hollow tubular element 800 for an inhaler article in accordance with an eight embodiment of the present disclosure. The hollow tubular element 800 comprises a peripheral portion 810 and a support element 830 formed integrally from the same sheet of paper. The sheet extends from a first end 833 of the sheet to a second end 834 of the sheet. The peripheral portion 810 is formed from parallel wound layers of the sheet such that a section of the peripheral portion 810 is formed from a single layer of the sheet and another section of the peripheral portion 810 is formed from two layers of the sheet.

The support element 830 extends from a first point 831 at the peripheral portion 810 across the hollow inner region to a second point 832 at the peripheral portion 810. In particular, the support element 830 depends from the peripheral portion 810 from both a first fold line and a second fold line of the sheet, wherein the first fold line resides at the first point 831 at the peripheral portion 810, and the second fold line resides at the second point 832 at the peripheral portion 810. The first point 831 at the peripheral portion 810 and the second point 832 at the peripheral portion 810 are about diametrically opposed to each other.

The portion of the sheet extending from the first end 833 of the sheet to the first point 831 at the peripheral portion 810, and the portion of the sheet extending from the second point 832 at the peripheral portion 810 to the second end 1034 of the sheet define the hollow inner region of the hollow tubular element 800. Accordingly, the peripheral portion 810 comprises the portion of the sheet extending from the first end 833 of the sheet to the first point 831 at the peripheral portion 810, and the portion of the sheet extending from the second point 832 at the peripheral portion 810 to the second end 834 of the sheet.

As shown in Figure 16, the support element 830 is substantially sinusoidal, when viewed from the upstream end of the hollow tubular element 800. The support element 830 comprises a plurality of peaks and troughs; in particular, the support element 830 comprises two peaks and three troughs. This increases the surface area of the hollow tubular element 800 that can be in contact with the capsule 9, for example, when the capsule 9 is being pierced by a piercing element. As such, this can increase the ability of the hollow tubular element 800 to prevent or restrict movement of the capsule 9, for example, when the capsule 9 is being pierced by a piercing element.

Figure 17 shows a cross-sectional view of the upstream end face of a hollow tubular element 900 for an inhaler article in accordance with a ninth embodiment of the present disclosure. The hollow tubular element 900 is generally the same as the hollow tubular element 800 of the eighth embodiment, with the exception that a second end of the sheet resides at the second point 932 at the peripheral portion 910. As such, there is no portion of the sheet extending from the second point 932 at the peripheral portion 910 to the second end of the sheet. Accordingly, the support element 930 does not depend from the peripheral portion 910 along a second fold line of the sheet, wherein the second fold line resides at the second point 932 of the peripheral portion 910. In addition, the peripheral portion 910 does not comprise a portion of the sheet extending from the second point 932 at the peripheral portion 910 to the second end of the sheet.

Furthermore, the hollow tubular element 900 differs from the hollow tubular element 800 in that the support element 930 is substantially s-shaped, when viewed from the upstream end of the hollow tubular element 900.

The support element 930 extends through the radial centre of the hollow tubular element 900.

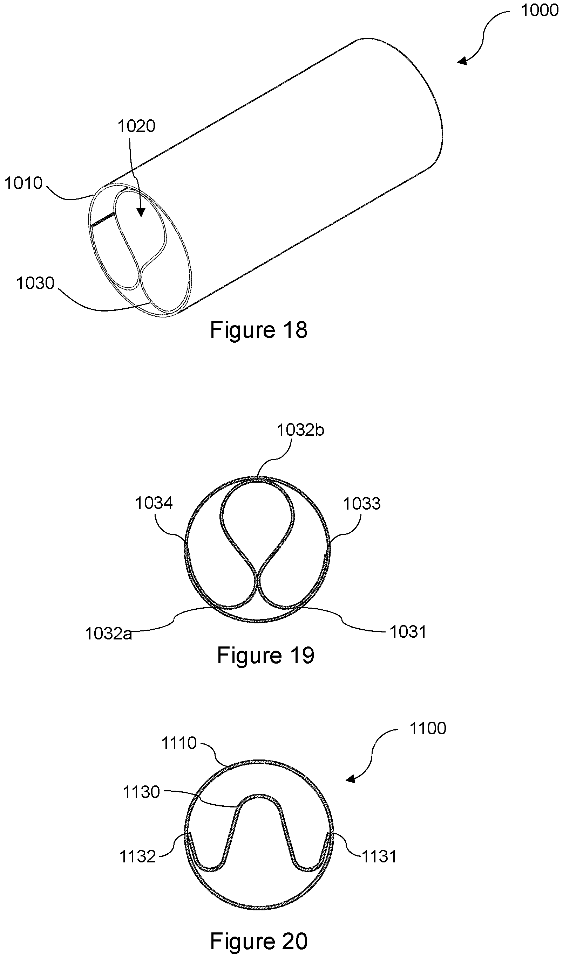

Figure 18 shows a perspective view of a hollow tubular element 1000 for an inhaler article in accordance with a tenth embodiment of the present disclosure.

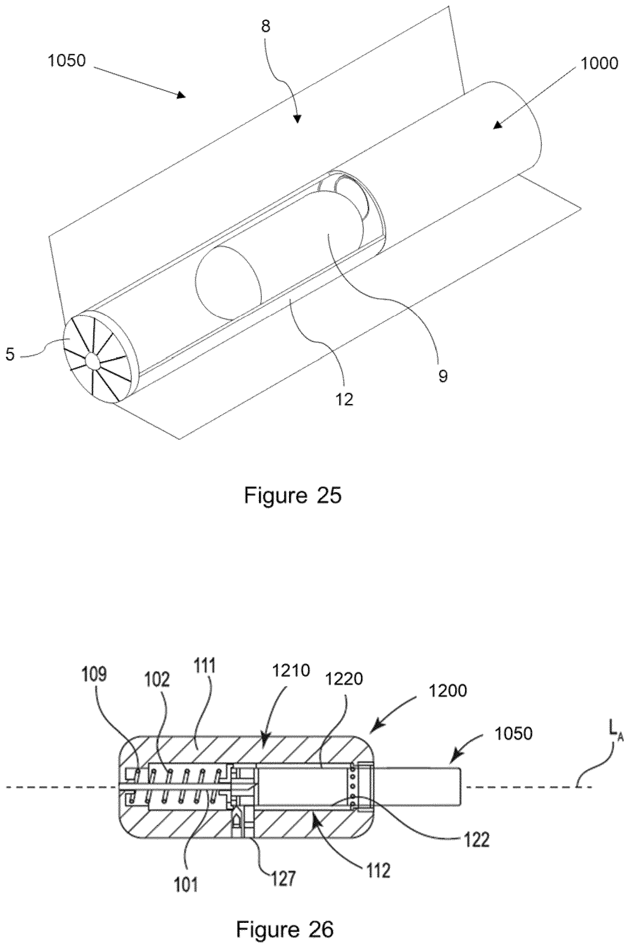

An inhaler article comprising the hollow tubular element 1000 is shown in Figure 25. The hollow tubular element 1000 comprises a peripheral portion 1010 which defines a hollow inner region 1020 of the hollow tubular element 1000. The hollow tubular element 1000 also comprises a support element 1030 formed from a sheet of paper. The peripheral portion 1010 comprises a tube that is distinct from the sheet which forms the support element 1030. That is, the tube is not integrally formed with the support element 1030.

As shown in Figure 19, a first end 1033 of the sheet is in contact with the tube up to a first point 1031 at the peripheral portion 1010, where it deflects away from the tube and into the hollow inner region 1020. A second end 1034 of the sheet is in contact with the tube up to a second point 1032a at the peripheral portion 1010, where it deflects away from the tube and into the hollow inner region 1020. As such, the support element 1030 extends from the first point 1031 at the peripheral portion 1010 across the hollow inner region 1020 to the second point 1032a at the peripheral portion 1010. In addition, the peripheral portion 1010 comprises: the tube, the portion of the sheet extending from the first end 1033 of the sheet to the first point 1031 at the peripheral portion 1010; and the portion of the sheet extending from the second point 1032a at the peripheral portion 1010 to the second end 1034 of the sheet.

The support element 1030 comprises a curved portion, when viewed from the upstream end of the hollow tubular element 100. In particular, the support element 1030 is substantially omega-shaped, when viewed from the upstream end of the hollow tubular element 1000. The support element 1030 is also in contact with the tube at a further point 1032b at the peripheral portion 1010. The support element 1030 divides the hollow inner region 1020 into four channels.

It will be appreciated that the portion of the sheet extending from the first point 1031 at the peripheral portion 1010 to the further point 1032b at the peripheral portion 1010 may be a first support element. In addition, the portion of the sheet extending from the further point 1032b at the peripheral portion 1010 to the second point 1032a at the peripheral portion 1010 may be a second support element. The first and second support elements divide the hollow inner region 1020 into four channels.

The sheet may be attached to the tube by an adhesive. In particular, the sheet may be attached to the tube at points where the sheet is in contact with the tube.

Figure 20 shows a cross sectional view of the upstream end face of a hollow tubular element 1100 for an inhaler article in accordance with an eleventh embodiment of the present disclosure. Similarly to the hollow tubular element 1000 of the tenth embodiment, the peripheral portion 1110 comprises a tube that is distinct from the sheet which forms the support element 1130. The support element 1130 is in contact with the peripheral portion 1110 at both a first point 1131 at the peripheral portion 1110 and a second point 1132 at the peripheral portion 1110. The support element extends from the first point 1131 at the peripheral portion 1110 across the hollow inner region to the second point 1132 at the peripheral portion 1110.

The support element 1130 has a wave profile, when viewed from the upstream end of the hollow tubular element 1100. In particular, the support element 1130 is substantially sinusoidal and comprises one peak and two troughs, when viewed from the upstream end of the hollow tubular element 1100.

Figure 21 shows a cross-sectional view of an upstream end face of a hollow tubular element 50 before a load F is applied, the hollow tubular element 50 before the load F has been applied has an original (undepressed) outer diameter $D_S$. Figure 21 also shows a cross-sectional view of an upstream end face of the same hollow tubular element 52 after applying a set load for a set duration (but with the load still applied), the hollow tubular element 52 after the set load has been applied for the set duration (but with the load still applied) has a (reduced) diameter $D_d$. The depression is $d = D_S - D_d$.

The apparatus for testing the hardness of the smoking articles filters is shown in Figures 22, 23 and 24.

Figure 22 is a perspective view of an apparatus 60, such as a DD60A Densimeter device, for determining the hardness of a hollow tubular element for an inhaler article. The apparatus 60 includes two parallel load applying rods 70 positioned over a support plate 80. The support plate 80 includes two parallel, spaced apart walls 82, with each wall 82 having ten equally spaced recesses. The recesses are arranged to prevent the hollow tubular elements 54 from contacting one another during testing.

As can be seen in Figure 22, ten identically designed hollow tubular elements 54 are aligned parallel in a plane, and placed on underlying cylindrical rods 84. The hollow tubular elements 54 extend between corresponding recesses in the walls 82 to hold the hollow tubular elements 54 in place. The underlying cylindrical rods 84 extend parallel to the walls 82. Each hollow tubular element 54 contacts the underlying rods 84 at two points, making for twenty total points of contact between the hollow tubular elements 54 to be tested and the underlying rods 84.

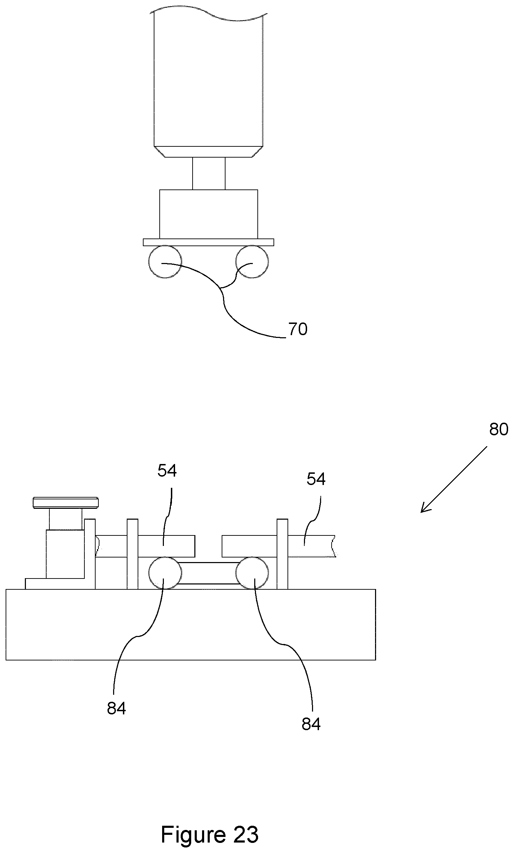

If the hollow tubular element 54 is too short and does not contact both underlying rods 84 or contacts the underlying rods 84 very close to the ends of the hollow tubular elements 54 to be tested, then it would appreciated that this could be achieved by using twenty hollow tubular elements 54 in a back-to-back configuration, such as that shown in Figure 23.

As shown, the concept of the DD60A Test is that the underlying cylindrical rods 84 contact the sample material 54 to be tested at twenty contact points. If the hollow tubular element 54 is sufficiently long to extend across the underlying rods 84, then the twenty contact points can be provided with ten samples (as shown in Figure 22). If the hollow tubular element 54 is not sufficiently long, then the twenty contact points can be provided with twenty samples, as shown in Figure 23.

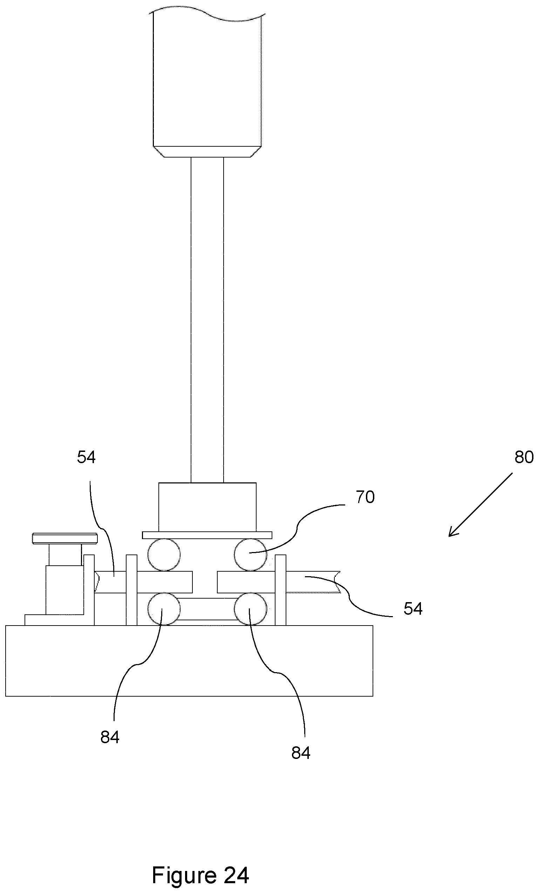

The apparatus is shown in Figure 23 in a first configuration, in which the two load applying cylindrical rods 70 are raised above and out of contact from the hollow tubular elements 54. To test the hardness of the hollow tubular element 54, the load applying cylindrical rods 70 are lowered to a second configuration, to come into contact with the hollow tubular elements 54, as shown in Figure 24. When in contact with the hollow tubular elements 54, the load applying rods 70 impart an overall load of 2kg across the twenty contact points of the hollow tubular element 54 for a duration of 20 seconds. After 20 seconds have elapsed (and with the load still being applied to the hollow tubular elements 54), the depression in the load applying cylindrical rods 70 across the hollow tubular element 54 is determined, and then used to calculate the hardness.

Figure 25 illustrates an inhaler article 1050 in accordance with a tenth embodiment of the present disclosure. The inhaler article 1050 of the tenth embodiment is generally the same as the inhaler article 10 of the first embodiment. However, the inhaler article 1050 of the tenth embodiment differs from the inhaler article 10 of the first embodiment in that the hollow tubular element 1000 of the inhaler article 1050 of the tenth embodiment is that shown in Figure 18.

Figure 26 illustrates an inhaler system 1200. The inhaler system 1200 includes an inhaler article 1050 and a separate holder 1210. The inhaler article 1050 may be received within the holder 1210 to activate or pierce the capsule 9 disposed within the inhaler article 1050. The inhaler article 1050 remains in the holder 1210 during use by the consumer. The holder 1210 is configured to induce swirling inhalation airflow entering the received inhaler article 1050. The holder 1210 is configured to fold back or breach or open the folded end 5 of the inhaler article 1050.

The inhaler system 1200 includes the inhaler article 1050 and the holder 1210. The inhaler article 1050 extends along an inhaler longitudinal axis LA. The holder 1210 includes a movable sleeve 1220 that retains the inhaler article 1050 received in the sleeve cavity 122.

The holder 1210 for the inhaler article 1050 includes a housing 111 comprising a housing cavity 112 for receiving the inhaler article1050 and the sleeve 1220 configured to retain the inhaler article 1050 within the housing cavity 112. The sleeve 1220 defines a sleeve cavity 122 and is movable within the housing cavity 112 along the longitudinal axis LA of the housing 111. The sleeve 1220 comprises a first open end 124 and a second opposing end 1226. The second opposing end 1226 of the sleeve 1220 is configured to allow air to enter the sleeve cavity 122. The second opposing end 1226 of the sleeve 1220 is configured to induce a swirl on the air entering the sleeve cavity 122.

The holder 1210 may include a piercing element 101 fixed to and extending from a housing inner surface 109. The piercing element 101 may be configured to extend through the second opposing end 1226 of the sleeve 1220 and into the sleeve cavity 122 along a longitudinal axis of the housing 111. The holder 1210 may include a spring element 102 configured to bias the sleeve 1220 away from the piercing element 101.

The sleeve 1220 may include an elongated slot extending along a longitudinal length of the sleeve 1220. The housing 111 may further comprise a pin 127 extending from an inner surface 109 of the housing cavity 112. The pin 127 may be configured to mate with the elongated slot.

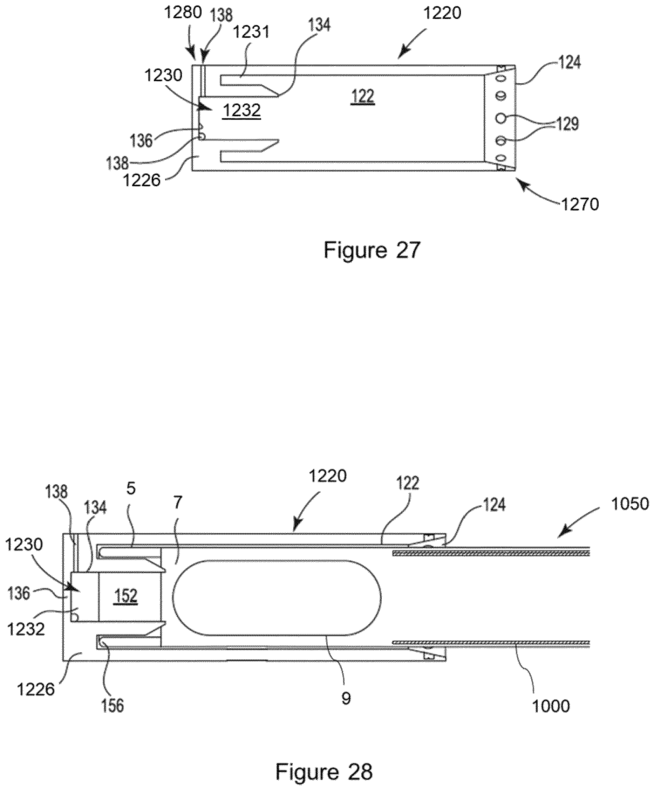

Figure 27 shows a schematic side sectional view of the sleeve 1220. The second opposing end 1226 of the sleeve 1220 comprises a sleeve tubular element 1230 defining a central passage 1232, an end surface 136 and an open end 134. The central passage 1232 in fluid communication with the sleeve cavity 122. The sleeve tubular element 1230 open end 134 may extend into the sleeve cavity 122. The sleeve tubular element 1230 includes at least one air inlet 138 allowing air to enter into the central passage 1232. The at least one air inlet 138 extends in a direction that is tangential to the central passage 1232.

The distal end 156 of the inhaler article 1250 may slide onto the sleeve tubular element 1230 as illustrated in Figure 28. The sleeve tubular element 1230 open end 134 changes the folded end 5 from a closed configuration to an open configuration allowing swirling or rotating inhalation air to flow directly into the inhaler article 1250 capsule cavity 7. Upon insertion of the inhaler article 1250 into the holder 1210, the sleeve tubular element 1230 open end 134 deforms and urges through the folded end 5 so that the sleeve tubular element 1230 extends into the received inhaler article 1250 hollow tube 12. The folded end 5 may be biased towards the longitudinal axis of the inhaler article in the open configuration so that the inhaler article 1250 grips onto the holder, thus holding the inhaler article 1250 in place in the holder 1210.

Inhalation air inlets 138 enter the sleeve tubular element 1230 at a tangent to the central passage 1232 and form swirling inhalation airflow to the capsule cavity 7 of a received inhaler article 1250. The swirling inhalation airflow flows along the capsule cavity 7 of a received inhaler article 1250 to induce capsule rotation and release particles into the inhalation airflow.

The sleeve tubular element 1230 may extend into the sleeve cavity 122 and forms an annular recess 1231 with the sleeve cavity 122 configured to receive a distal end 156 of an inhaler article 1250. The projection formed by the sleeve tubular element 1230 slides into the inhaler article 1250 capsule cavity 7. The sleeve tubular element 1230 is configured here to extend into a distal end 156 of an inhaler article 1250 received within the sleeve cavity 122.

The sleeve tubular element 1230 may extend into the sleeve cavity 122 about 5 mm and have an outer diameter of about 6.5 mm and an inner diameter of about 4 mm. The capsule cavity 7 of a received inhaler article 1250 may have an inner diameter of about 6.6 mm to provide an interference fit with the sleeve tubular element 1230 and annular recess 1231.

The sleeve 1220 defines a first air inlet zone 1270 comprising at least one air aperture 129 through the sleeve 1220. The first air inlet zone 1270 proximate to the first open end 124 of the sleeve 1220. The first air inlet zone 1270 is configured to allow air to flow to an airflow channel formed between the sleeve 1220 and the housing 111. The sleeve comprises a second air inlet zone 1280 in downstream from the first air inlet zone 1270. The second air inlet zone 1280 comprising the second opposing end 1226 of the sleeve 1220 configured to allow air to enter the sleeve cavity 122. The second air inlet zone 1280 comprising at least one air aperture or air inlet 138 through the sleeve 1220 and into the sleeve tubular element 1230 having a central passage 1232.

Figure 28 shows a schematic side sectional view of the inhaler article 1250 of Figure 18 received in the sleeve 1220 of Figure 27. As illustrated in Figure 28, the capsule cavity 7 of the inhaler article 1250 aligns and mates with and extends into the central passage 1232 of the sleeve tubular element 1230. The sleeve tubular element 1230 forms the upstream end of the capsule cavity 7. The folded end 5 is opened up back on to the capsule cavity 7 sidewall and providing an interference fit within the annular recess 1231.

**Claims**

1. An inhaler article (10) comprising:

   a cavity (7);
   a capsule (9) located in the cavity, the capsule (9) containing dry powder; and
   a hollow tubular element (100) disposed downstream of the capsule (9),
   wherein the hollow tubular element (100) comprises:

a peripheral portion (110) defining a hollow inner region (120) of the hollow tubular element (100); and

a support element (130) formed from a sheet and extending from a first point (131) at the peripheral portion (110) across the hollow inner region (120) to a second point (132) at the peripheral portion (110).

2. An inhaler article (10) according to claim 1, wherein the peripheral portion (110) is formed from a sheet.

3. An inhaler article (10) according to claim 2, wherein the peripheral portion (110) and the support element (130) are integrally formed from a sheet.

4. An inhaler article (10) according to claim 3, wherein the peripheral portion (110) and the support element (130) are formed from separate sheets.

5. An inhaler article (10) according to any one of the preceding claims, wherein the first point (131) at the peripheral portion (110) and the second point (132) at the peripheral portion (110) are spaced apart from each other.

6. An inhaler article (10) according to any one of the preceding claims, wherein the support elements (130) depends from the peripheral portion along a first fold line (141) of the sheet, wherein the first fold line (141) resides at the first point (131) at the peripheral portion (110).

7. An inhaler article (10) according to claim 6, wherein the support element (130) depends from the peripheral portion (110) along a second fold line (142) of the sheet, wherein the second fold line (142) resides at the second point (132) at the peripheral portion (110).

8. An inhaler article (10) according to any one of the preceding claims, wherein a cross section of the support element (730) comprises a curved portion.

9. An inhaler article (10) according to claim 8, wherein a cross section of the support element (1030) is substantially omega-shaped.

10. An inhaler article (10) according to any one of the preceding claims, wherein the support element (630) divides the hollow inner region (620) into a plurality of channels.

11. An inhaler article (10) according to any one of the preceding claims, wherein the support element (130) is spaced apart from the from the radial centre of the hollow tubular element (100) by a distance of between about 5 percent and about 90 percent of the radius of the hollow tubular element (100).

12. An inhaler article (10) according to any one of the preceding claims, wherein the hollow tubular element (100) is configured to sustain a force of up to 15 Newtons being applied to its upstream end without deforming substantially.

13. An inhaler article (10) according to any one of the preceding claims, wherein the Young's modulus of the material of the hollow tubular element (100) is greater than 10 MPa.

14. An inhaler article (10) according to any one of the preceding claims, wherein the hollow tubular element (100) extends from the cavity (7) to the downstream end of the inhaler article (10).

15. An inhaler system comprising an inhaler article (10) according to any one of the preceding claims and a holder for receiving the inhaler article, the holder comprising:

a housing defining a housing cavity configured to receive the inhaler article (10); and a piercing element (101) configured to extend into the housing cavity and pierce the capsule (9) of the inhaler article (10).

**Patentansprüche**

1. Inhalatorartikel (10), aufweisend:

einen Hohlraum (7);
eine in dem Hohlraum angeordnete Kapsel (9), wobei die Kapsel (9) Trockenpulver enthält; und
ein hohles rohrförmiges Element (100), das der Kapsel (9) nachgelagert angeordnet ist, wobei das hohle rohrförmige Element (100) umfasst:

einen Umfangsabschnitt (110), der eine hohle Innenregion (120) des hohlen rohrförmigen Elements (100) definiert; und
ein aus einer Bahnware gebildetes und sich von einem ersten Punkt (131) an dem Umfangsabschnitt (110) über die hohle Innenregion (120) zu einem zweiten Punkt (132) an dem Umfangsabschnitt (110) erstreckendes Auflageelement (130).

2. Inhalatorartikel (10) nach Anspruch 1, wobei der Umfangsabschnitt (110) aus einer Bahnware gebildet ist.

3. Inhalatorartikel (10) nach Anspruch 2, wobei der Umfangsabschnitt (110) und das Auflageelement (130) einstückig aus einer Bahnware gebildet sind.

4. Inhalatorartikel (10) nach Anspruch 3, wobei der Umfangsabschnitt (110) und das Auflageelement (130) aus separaten Bahnwaren gebildet sind.

5. Inhalatorartikel (10) nach einem beliebigen der vorhergehenden Ansprüche, wobei der erste Punkt (131) an dem Umfangsabschnitt (110) und der zweite Punkt (132) an dem Umfangsabschnitt (110) voneinander beabstandet sind.

6. Inhalatorartikel (10) nach einem beliebigen der vorhergehenden Ansprüche, wobei die Auflageelemente (130) von dem Umfangsabschnitt entlang einer ersten Falzlinie (141) der Bahnware abhängen, wobei sich die erste Falzlinie (141) an dem ersten Punkt (131) an dem Umfangsabschnitt (110) befindet.

7. Inhalatorartikel (10) nach Anspruch 6, wobei das Auflageelement (130) von dem Umfangsabschnitt (110) entlang einer zweiten Falzlinie (142) der Bahnware abhängt, wobei sich die zweite Falzlinie (142) an dem zweiten Punkt (132) an dem Umfangsabschnitt (110) befindet.

8. Inhalatorartikel (10) nach einem beliebigen der vorhergehenden Ansprüche, wobei ein Querschnitt des Auflageelements (730) einen gekrümmten Abschnitt aufweist.

9. Inhalatorartikel (10) nach Anspruch 8, wobei ein Querschnitt des Auflageelements (1030) im Wesentlichen omegaförmig ist.

10. Inhalatorartikel (10) nach einem beliebigen der vorhergehenden Ansprüche, wobei das Auflageelement (630) die hohle Innenregion (620) in eine Vielzahl von Kanälen unterteilt.

11. Inhalatorartikel (10) nach einem beliebigen der vorhergehenden Ansprüche, wobei das Auflageelement (130) von der radialen Mitte des hohlen rohrförmigen Elements (100) um eine Distanz zwischen etwa 5 Prozent und etwa 90 Prozent des Radius des hohlen rohrförmigen Elements (100) beabstandet ist.

12. Inhalatorartikel (10) nach einem beliebigen der vorhergehenden Ansprüche, wobei das hohle rohrförmige Element (100) ausgelegt ist, einer auf sein vorgelagertes Ende ausgeübten Kraft von bis zu 15 Newton standzuhalten, ohne sich wesentlich zu verformen.

13. Inhalatorartikel (10) nach einem beliebigen der vorhergehenden Ansprüche, wobei der Elastizitätsmodul des Materials des hohlen rohrförmigen Elements (100) größer als 10 MPa ist.

14. Inhalatorartikel (10) nach einem beliebigen der vorhergehenden Ansprüche, wobei sich das hohle rohrförmige Element (100) von dem Hohlraum (7) zu dem nachgelagerten Ende des Inhalatorartikels (10) erstreckt.

15. Inhalatorsystem, umfassend einen Inhalatorartikel (10) nach einem beliebigen der vorhergehenden Ansprüche und einen Halter zum Aufnehmen des Inhalatorartikels, der Halter umfassend:
ein Gehäuse, das einen zum Aufnehmen des Inhalatorartikels (10) ausgelegten Gehäusehohlraum definiert; und ein zum sich Erstrecken in den Gehäusehohlraum und zum Durchstechen der Kapsel (9) des Inhalatorartikels (10) ausgelegtes Durchstechelement (101).

## Revendications

1. Article inhalateur (10) comprenant :

une cavité (7) ;
une capsule (9) située dans la cavité, la capsule (9) contenant de la poudre sèche ; et
un élément tubulaire creux (100) disposé en aval de la capsule (9),
dans lequel l'élément tubulaire creux (100) comprend :

une portion périphérique (110) définissant une région intérieure creuse (120) de l'élément tubulaire creux (100) ; et
un élément de support (130) formé à partir d'une feuille et s'étendant depuis un premier point (131) au niveau de la portion périphérique (110) sur la région intérieure creuse (120) jusqu'à un deuxième point (132) au niveau de la portion périphérique (110).

2. Article inhalateur (10) selon la revendication 1, dans lequel la portion périphérique (110) est formée à partir d'une feuille.

3. Article inhalateur (10) selon la revendication 2, dans lequel la portion périphérique (110) et l'élément de support (130) sont formés d'un seul bloc à partir d'une feuille.

4. Article inhalateur (10) selon la revendication 3, dans lequel la portion périphérique (110) et l'élément de support (130) sont formés à partir de feuilles séparées.

5. Article inhalateur (10) selon l'une quelconque des revendications précédentes, dans lequel le premier point (131) au niveau de la portion périphérique (110) et le deuxième point (132) au niveau de la portion périphérique (110) sont espacés l'un par rapport à l'autre.

6. Article inhalateur (10) selon l'une quelconque des revendications précédentes, dans lequel l'élément de support (130) dépend de la portion périphérique le long d'une première ligne de pliage (141) de la feuille, dans lequel la première ligne de pliage (141) se trouve au niveau du premier point (131) au niveau de la portion périphérique (110).

7. Article inhalateur (10) selon la revendication 6, dans lequel l'élément de support (130) dépend de la portion périphérique (110) le long d'une deuxième ligne de pliage (142) de la feuille, dans lequel la deuxième ligne de pliage (142) se trouve au niveau du deuxième point (132) au niveau de la portion périphérique (110).

8. Article inhalateur (10) selon l'une quelconque des revendications précédentes, dans lequel une coupe transversale de l'élément de support (730) comprend une portion incurvée.

9. Article inhalateur (10) selon la revendication 8, dans lequel une coupe transversale de l'élément de support (1030) est sensiblement en forme d'oméga.

10. Article inhalateur (10) selon l'une quelconque des revendications précédentes, dans lequel l'élément de support (630) divise la région intérieure creuse (620) en une pluralité de canaux.

11. Article inhalateur (10) selon l'une quelconque des revendications précédentes, dans lequel l'élément de support (130) est espacé du centre radial de l'élément tubulaire creux (100) d'une distance d'entre environ 5 pour cent et environ 90 pour cent du rayon de l'élément tubulaire creux (100).

12. Article inhalateur (10) selon l'une quelconque des revendications précédentes, dans lequel l'élément tubulaire creux (100) est configuré pour supporter une force allant jusqu'à 15 Newtons qui est appliquée à son extrémité amont sans déformation sensible.

13. Article inhalateur (10) selon l'une quelconque des revendications précédentes, dans lequel le module de Young de la matière de l'élément tubulaire creux (100) est supérieur à 10 MPa.

14. Article inhalateur (10) selon l'une quelconque des revendications précédentes, dans lequel l'élément tubulaire creux (100) s'étend de la cavité (7) à l'extrémité aval de l'article inhalateur (10).

15. Système inhalateur comprenant un article inhalateur (10) selon l'une quelconque des revendications précédentes et un support destiné à recevoir l'article inhalateur, le support comprenant :

un logement définissant une cavité de logement configurée pour recevoir l'article inhalateur (10) ; et
un élément de perçage (101) configuré pour s'étendre dans la cavité de logement et percer la capsule (9) de l'article inhalateur (10).

Figure 1

Figure 2

Figure 3

Figure 4A

Figure 4B

Figure 4C

Figure 5

Figure 6

330    300

380    370

Figure 7

431   432    400

430

Figure 8

500

530

580    570

Figure 9

EP 4 262 942 B1

Figure 10

Figure 11A

Figure 11B

Figure 12A

Figure 12B

39

Figure 13

Figure 14

Figure 15

Figure 16

Figure 17

Figure 18

Figure 19

Figure 20

Figure 21

Figure 22

70

80

54

54

84

84

Figure 23

Figure 24

Figure 25

Figure 26

Figure 27

Figure 28

**REFERENCES CITED IN THE DESCRIPTION**

**Patent documents cited in the description**

- WO 2020178714 A1